# EUROPEAN PATENT SPECIFICATION

(11) **EP 1 791 417 B1**
(45) Date of publication and mention of the grant of the patent: **03.10.2012**
(21) Application number: 05770849.7
(22) Date of filing: 05.08.2005
(51) Int. Cl.: C12N 15/82, A01H 5/00

(54) **Polynucleotides, DNA constructs and methods for the alteration of plant lignin content and/or composition**
Polynukleotide, DNA-Konstrukte und Verfahren für die Modifikation des Ligningehalts von Pflanzen, und/oder deren Ligninzusammensetzung
Polynucleotides, structures d'ADN et procédés destines a la modification de la teneur des plantes en lignine et/ou de leur composition

(30) Priority: 18.08.2004 US 602440 P
(43) Date of publication of application: 06.06.2007
(60) Divisional application: 10189451.7
(73) Proprietor: Alellyx S.A., 13069-380 Campinas SP (BR)
(72) Inventor: PAPES, Fabio, CEP-13069-380 Campinas/SP (BR); ARRUDA, Paulo, CEP-13069-380 Campinas/SP (BR)
(74) Representative: Plougmann & Vingtoft A/S
(86) International application number: PCT/BR2005/000162
(87) International publication number: WO 2006/017920

(56) References cited:
- WO-A-95/14092
- US-A1- 2003 163 851
- WAIE BHAVNA ET AL: "Effect of increased polyamine biosynthesis on stress responses in transgenic tobacco by introduction of human S-adenosylmethionine gene." PLANT SCIENCE (OXFORD), vol. 164, no. 5, May 2003 (2003-05), pages 727-734, XP002481340 ISSN: 0168-9452
- ROY MALABIKA ET AL: "Overexpression of S-adenosylmethionine decarboxylase gene in rice increases polyamine level and enhances sodium chloride-stress tolerance." PLANT SCIENCE (OXFORD), vol. 163, no. 5, November 2002 (2002-11), pages 987-992, XP002481341 ISSN: 0168-9452
- THU-HANG PHAM ET AL: "Expression of a heterologous S-adenosylmethionine decarboxylase cDNA in plants demonstrates that changes in S-adenosyl-L-methionine decarboxylase activity determine levels of the higher polyamines spermidine and spermine" PLANT PHYSIOLOGY (ROCKVILLE), vol. 129, no. 4, August 2002 (2002-08), pages 1744-1754, XP002481342 ISSN: 0032-0889
- DATABASE EMBL [Online] 3 April 1997 (1997-04-03), "Nicotiana tabacum S-adenosylmethionine decarboxylase mRNA, complete cds." XP002481345 retrieved from EBI accession no. EMBL:U91924 Database accession no. U91924
- DATABASE EMBL [Online] 27 January 1998 (1998-01-27), "Nicotiana tabacum S-adenosylmethionine decarboxylase gene, complete cds." XP002481346 retrieved from EBI accession no. EMBL:AF033100 Database accession no. AF033100
- SHEN BO ET AL: "High free-methionine and decreased lignin content result from a mutation in the Arabidopsis S-adenosyl-L-methionine synthetase 3 gene" PLANT JOURNAL, vol. 29, no. 3, February 2002 (2002-02), pages 371-380, XP002481343 ISSN: 0960-7412
- QUAN YI ET AL: "Genetic manipulation of polyamine metabolism in poplar II: Effects on ethylene biosynthesis." PLANT PHYSIOLOGY AND BIOCHEMISTRY (PARIS), vol. 40, no. 11, November 2002 (2002-11), pages 929-937, XP002481344 ISSN: 0981-9428
- BAGNI N. ET AL.: 'Biosynthesis, oxidation and conjugation of aliphatic polyamines in higher plants' AMINO ACIDS vol. 20, 2001, pages 301 - 317, XP003013480
- BAUCHER ET AL.: 'Lignin: Genetic Engineering and Impact on Pulping' CRITICAL REVIEWS IN BIOCHEMISTRY AND MOLECULAR BIOLOGY vol. 38, 2003, pages 305 - 350, XP008081545

## Description

### FIELD OF INVENTION

The present invention relates to the field of molecular biology and the regulation of protein synthesis through the introduction of foreign genes into plant cells, preferably into their genomes. More specifically, the method relates to the modification of plant lignin content and/or composition in a plant cell by the introduction of a foreign gene encoding an active S-adenosylmethionine decarboxylase (SAMdc) enzyme.

### BACKGROUND

Lignin is one of the major products of the general phenylpropanoid pathway, and is one of the most abundant organic molecules in the biosphere. Lignin accounts for 20-30% of the dry weight of trees and is primarily deposited in the cell walls of supporting and conductive tissues, providing rigidity to the wood and structural integrity to tracheary elements (Baucher et al., Crit. Rev. Biochem. Mol. Biol. 38:305-50 (2003)). Lignification also occurs after microbial infection or wounding to protect tissues from pathogen penetration (Baucher et al., Crit. Rev. Biochem. Mol. BioL 38:305-50 (2003); Boerjan et al., Annu. Rev. Plant. Biol. 54:519-46 (2003); Crawford, (1981) Lignin Biodegradation and Transformation, New York: John Wiley and Sons).

Lignin's resistance to degradation has a significant impact on the utilization of lignocellulosic plant materials: it decreases the digestibility of animal forage crops (Cymbaluk et al., Br. J. Nutr. 29:1-12 (1973)) and must be removed during pulping and papermaking, which requires the use of environmentally hazardous chemicals (Baucher et al., Crit. Rev. Biochem. Mol. Biol. 38:305-50 (2003); Whetten et al., Forest Ecol. Management 43, 301, (1991)). Therefore, improvement in the digestibility of forage plants or reductions in the amount of chemicals used for kraft pulping would be of great value (Baucher et al., Crit. Rev. Biochem. Mol. Biol. 38:305-50 (2003); Sederoff et al., Genetic Engineering of Plant Secondary Metabolism, New York Plenum Press (1994)).

Lignins are the result of dehydrogenative polymerization of monolignols, notably p-coumaryl, coniferyl and synapyl alcohols (reviewed in Boerjan et al., Annu. Rev. Plant. Biol. 54:519-46 (2003)). Different plant species or cell types harbor lignin polymers composed of varying proportions of these three monolignols. For example, gymnosperm lignin is primarily composed of guaiacyl (coniferyl-derived) units, whereas angiosperm dicot lignin is primarily composed of guaiacyl and syringyl (synapyl-derived) units. Grass lignin, on the other hand, is a mixture of guaiacyl, syringyl and p-hydroxylphenyl (cournaryl-derived) units (Campbell and Sederoff, Plant. Physiol. 110:3-13 (1996)). It is well known that the monomeric composition of lignin has a significant effect on its chemical degradation during industrial pulping (Baucher et al., Plant Physiol. 112: 1479-1490 (1996); O'Connell et al., Transgenic Res. 11: 495-503 (2002); Baucher et al., Critical Reviews in Biochemistry and Molecular Biology, 38:305-350 (2003)).

Monolignols are synthesized through the phenylpropanoid biosynthetic pathway. The chemical structures of the various monolignols, for example, p-coumaric acid, ferulic acid and sinapic acid, differ typically in the number and position of methoxy groups on their aromatic ring (Boerjan et al., Annu. Rev. Plant BioL 54: 519-546 (2003)). Such methoxy groups originate from S-adenosylmethionine (SAM), an important substrate in many of the reactions catalyzed by enzymes in the phenylpropanoid pathway (Boerjan et al., Anni. Rev. Plant Biol. 54: 519-546 (2003)). SAM is synthesized from methionine by the action of one or more SAM synthetase isoforms and acts as a cofactor in many processes in plant cells besides lignification, such as DNA methylation and ethylene, biotin and polyamine biosynthesis (Ravanel et al., Proc. Natl. Acad., Sci. USA 95: 7805-7812 (1998)).

Since SAM represents a fundamental compound for lignin biosynthesis, reduction of lignin content in a plant with impaired SAM availability in lignin-synthesizing tissues is envisioned, which could be obtained either by affecting the synthesis of SAM or by increasing its degradation. Impairment in the synthesis of SAM in a transgenic plant can be carried out through silencing of gene(s) encoding enzymes involved in SAM synthesis, as for example SAM synthetase. However, the silencing effect thus obtained could easily spread to the entire plant, since the gene encoding SAMdc is highly expressed in several plant tissues. Lower SAM synthetase levels would in turn translate into reduction of SAM in tissues other than those involved in lignin deposition, which could then affect many other metabolic processes, leading to undesirable plant phenotypes.

Alternatively, increasing the level of SAM degradation or the rate of its conversion into another compound would also result in decreased SAM availability. SAM is converted to decarboxylated SAM (dSAM), a precursor in the polyamine biosynthesis pathway, by the action of SAM decarboxylase (SAMdc; EC. number 4.1.1.50) (Malmberg et al., Crit. Rev. Plant Sci. 17:199-224 (1998)). Therefore, a plausible way to reduce SAM levels in a plant cell and hence its lignin content is via the overexpression of SAMdc in lignin-synthesizing tissues. Vascular tissues, which are the major lignin deposition sites in angiosperms, have low SAMdc gene expression levels, leading to the assumption that specific and localized SAMdc gene overexpression in these tissues would affect the local concentrations of SAM and thus impair lignin biosynthesis and deposition rates.

Reduction of lignin content and/or alteration of lignin composition is desirable in that it would reduce pulping-derived pollution and improve papermaking processes. It is therefore of interest to develop methods that allow the modification of lignin content and/or the composition of a plant, e.g., a cell, and/or tissue, to produce a plant having altered lignin content.

### SUMMARY

The present invention utilizes isolated nucleic acid molecules comprising the nucleotide sequences of SEQ ID NOS.: 1, 3, 5, 7, 9, 11, 13, 15, 17 and 19, which encode active SAMdc enzymes, wherein the enzymes have amino acid sequences which correspond to SEQ ID NOs.: 2, 4, 6, 8, 10, 12, 14, 16 and 18, and wherein the proteins encompass amino acid substitutions, additions and deletions that do not alter the function of the SAMdc enzyme.

The invention utilizes nucleic acid molecules likely to cause altered lignin content in a transformed plant, as well as molecules comprising fragments corresponding to cDNAs or genomic sequences, in part or as a whole, encoding active SAMdc enzymes. An important aspect is the use of DNA constructs wherein a SAMdc gene is operably linked to one or more suitable promoter sequences, which allow the SAMdc gene to be expressed in certain cell types, organs or tissues so as to exclusively alter the transformed plant's lignin content without affecting its normal development or physiology.

Also provided is a method for altering the activity of SAMdc in a plant by means of transforming this plant with a DNA construct wherein a SAMdc gene is operably linked to suitable promoter sequences that allow for the SAMdc gene to be expressed in certain cell types, organs or tissues so as to exclusively alter the plant's lignin content.

Methods for altering the activity of SAMdc enzyme include (i) plant transformation techniques such as particle bombardment (Klein et al., Biotechnology, 4: 583-590 (1993)), *Agrobacterium*-mediated transformation (Bechtold et al., C. R. Acad. Sci. Paris 316:1194-1199 (1993); Bent et al., Mol. Gen. Genet. 204:383-396 (1986)), among others (Paszowski et al., EMBO J. 3: 2717-2722 (1984); Sagi et aL, Plant Cell Rep. 13: 262-266 (1994)); (ii) regeneration of transformed plants from transformed cells, tissues or organs or from transformed seeds; (iii) selecting transformed plants which have a phenotype characterized by (1) an ability of the whole plant to accumulate less compounds derived from monolignols and (2) an reduced lignin content relative to an untransformed host plant.

The disclosure also provides a plant characterized by reduced lignin content and/or altered lignin composition compared to a wild-type plant. Plants include, for example, gymnosperms and woody trees such as eucalyptus, poplar and pine. The plant is a genetically engineered plant. More preferably, the plant exhibits increased activity of the enzyme SAMdc, as compared to the activity of SAMdc in the wild-type plant. In an especially preferred disclosure, a plant is genetically engineered using transgenic technology to effect an increase in the activity of a SAMdc enzyme in certain cell types, organs or tissues, by means of using a DNA construct wherein a SAMdc gene is operably linked to suitable promoter sequences so as to exclusively alter the plant's lignin content Accordingly, a preferred plant contains at least one exogenous nucleic acid comprising a nucleotide sequence that is at least a portion of a SAMdc gene such that, when the exogenous nucleic acid is present, the activity of SAMdc is increased.

Methods for making the plants of the invention are also provided. Plants are genetically engineered to increase the activity of SAMdc, an enzyme involved in the conversion of S-adenosylmethionine into decarboxylated S-adenosyonethionine, in specific plant cell types, tissues or organs. The disclosure provides methods for the production of genetically engineered plants that includes transecting a plant cell with at least one exogenous nucleic acid (comprising a SAMdc gene operably linked to suitable promoter sequences) associated with increased activity of at least one SAMdc enzyme in specific plant cell types, tissues or organs as a means to specifically impair lignin biosynthesis, followed by growing the transfected plant cell into the genetically engineered plant having reduced lignin content in comparison to the lignin content of a comparable wild-type plant.

### BRIEF DESCRIPTION OF THE DRAWINGS

**FIG. 1** illustrates the lignin biosynthesis pathway, showing in details the compounds, reactions and enzymes involved in synthesis of monolignols, the building blocks of the three kinds of lignin, represented at the bottom of the picture as p-hydroxyphenyl, guaiacyl and syringyl lignins.

**FIG. 2** schematically illustrates the plant expression plasmidial vector pALELLYX-ATG comprising a cambium/xylem preferred promoter driving the expression of a SAMdc nucleotide sequence

**FIG. 3** schematically illustrates the plant expression plasmidial vector pALELLYX-Nt comprising a cambium/xylem preferred promoter driving the expression of a SAMdc nucleotide sequence from *Nicotiana tabacum* (tobacco).

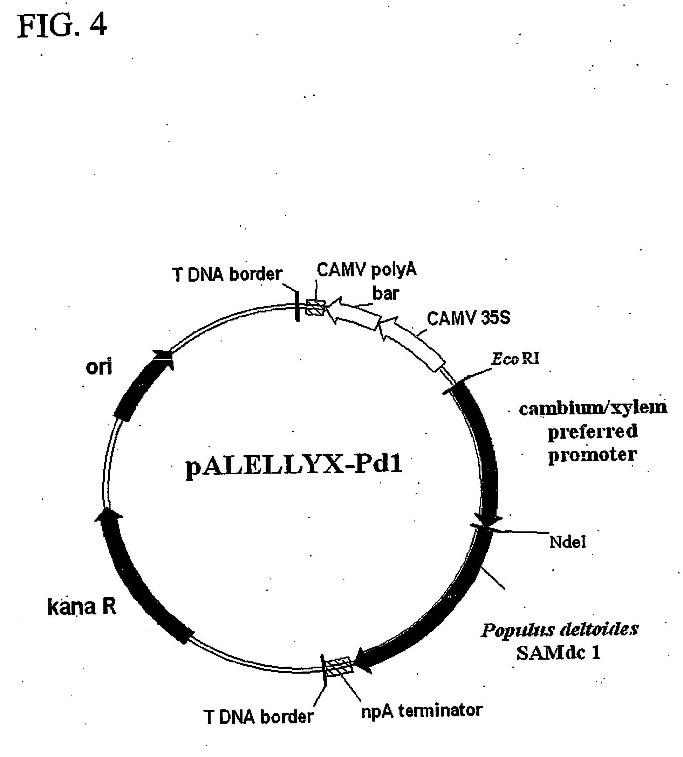

**FIG. 4** schematically illustrates the plant expression plasmidial vector pALELLYX-Pd1 comprising a cambium/xylem preferred promoter driving the expression of SAMdc nucleotide sequence coding for SAMdc 1 from *Populus deltoides.*

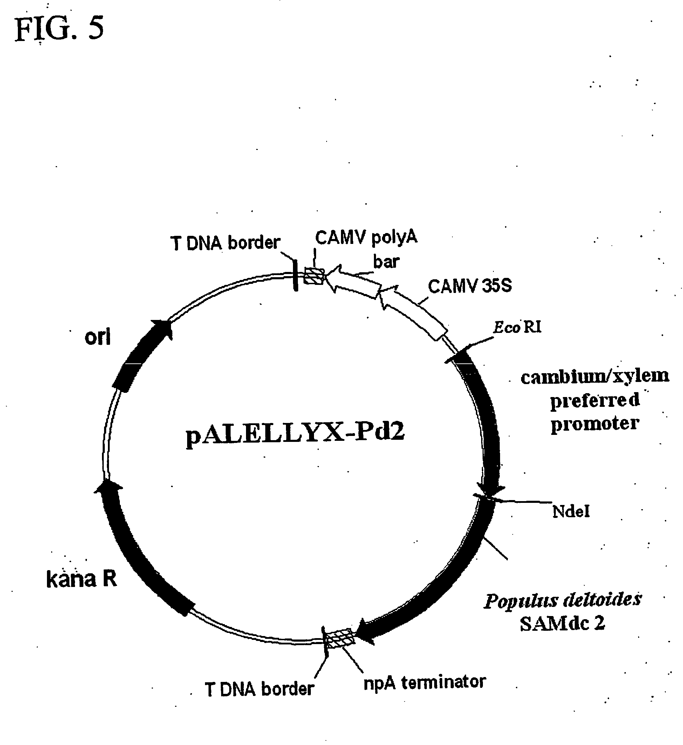

**FIG. 5** schematically illustrates the plant expression plasmidial vector pALELLYX-Pd2 comprising a cambium/xylem preferred promoter driving the expression of SAMdc nucleotide sequence coding for SAMdc 2 from *Populus deltoides.*

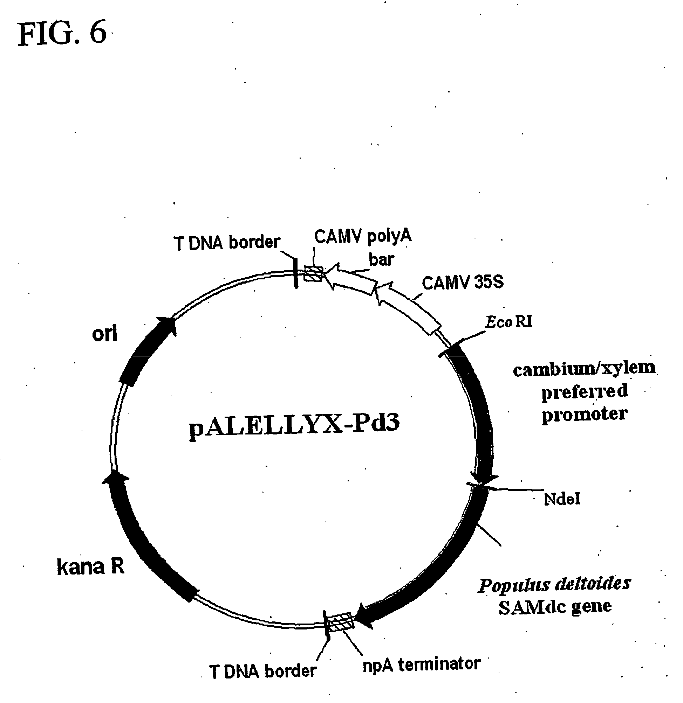

**FIG. 6** schematically illustrates the plant expression plasmidial vector pALELLYX-Pd3 comprising a cambium/xylem preferred promoter upstream of a SAMdc genomic sequence from *Populus deltoides.*

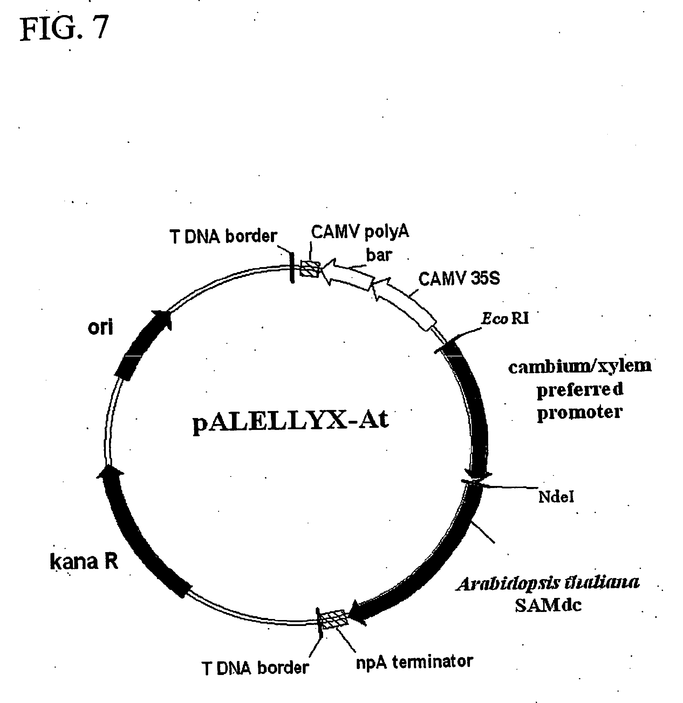

**FIG. 7** schematically illustrates the plant expression plasmidial vector pALELLYX-At comprising a cambium/xylem preferred promoter driving the expression of a SAMdc nucleotide sequence from *Arabidopsis thaliana* (thale cress).

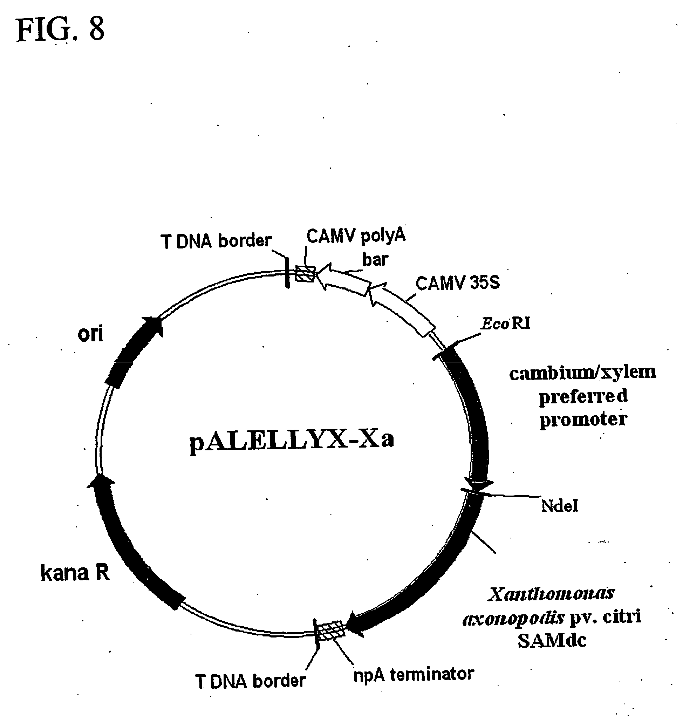

**FIG. 8** schematically illustrates the plant expression plasmidial vector pALELLYX-Xa comprising a cambium/xylem preferred promoter upstream of an open reading frame coding for SAMdc from the plant pathogen *Xanthomonas axonopodis*

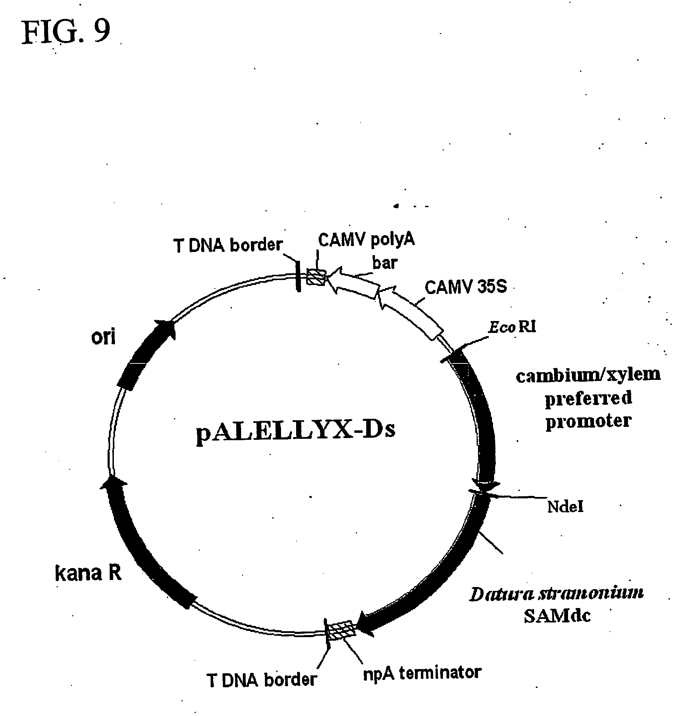

**FIG. 9** schematically illustrates the plant expression plasmidial vector pALELLYX-Ds comprising a cambium/xylem preferred promoter driving the expression of a SAMdc nucleotide sequence from *Datura stramonium.*

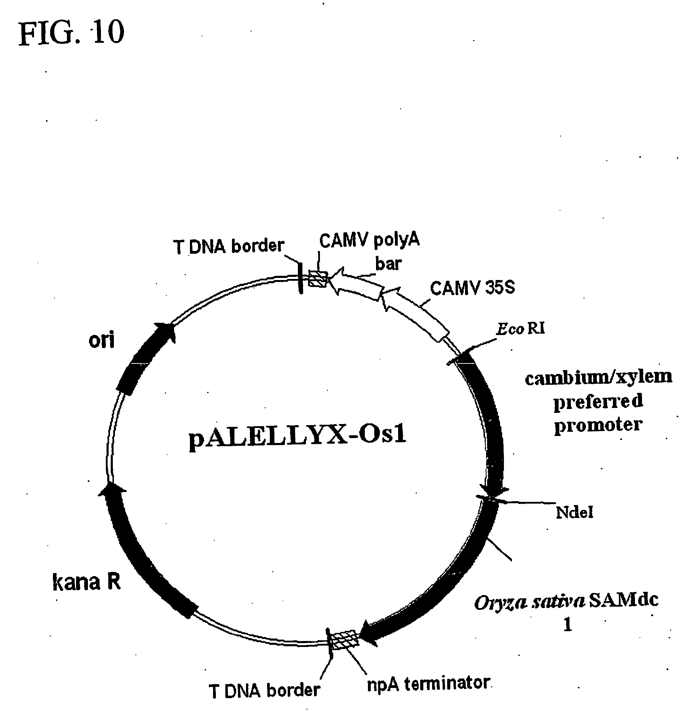

**FIG. 10** schematically illustrates the plant expression plasmidial vector pALELLYX-Os1 comprising a cambium/xylem preferred promoter driving the expression of a nucleotide sequence coding for SAMdc 1 from *Oryza sativa* (rice).

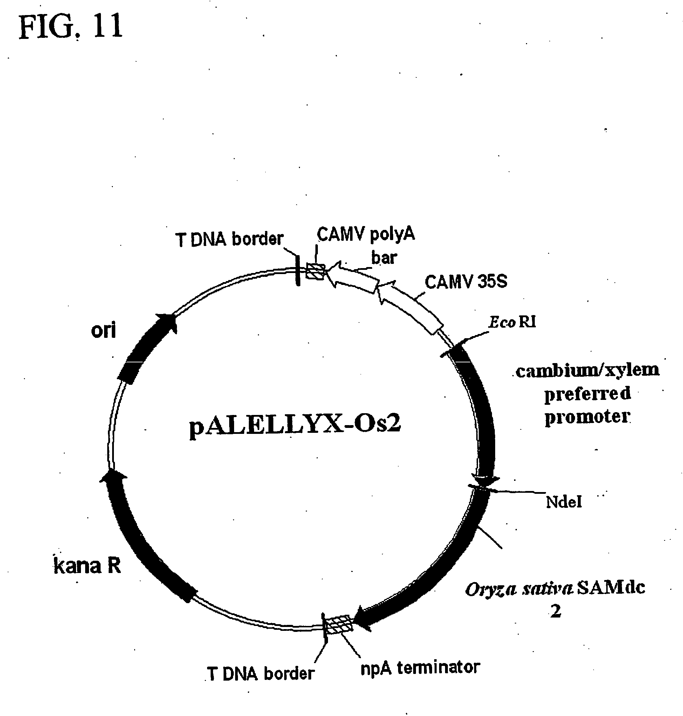

**FIG. 11** schematically illustrates the plant expression plasmidial vector pALELLYX-Os2 comprising a cambium/xylem preferred promoter driving the expression of a nucleotide sequence coding for SAMdc 2 from *Oryza sativa* (rice).

**FIG. 12** shows hand-sectioned unfixed stems (rosette base level) of *Arabidopsis thaliana* stained with the lignin-specific dye phloroglucinol. (A) Control non-transgenic plant TUB-SAMdc-Nt22; (B) Transgenic plant TUB-SAMdc-Nt12, which exhibits a high transgene expression level (FIG. 13); (C) Control non-transgenic plant TUB-SAMdc-Nt01; (D) Transgenic plant TUB-SAMdc-Nt09. Insets represent lower magnification images of the respective stem cuts.

**FIG. 13** exhibits a semi-quantitative RT-PCR experiment showing the transgene expression level (SAMdc) relative to the expression level of a control gene (APTR, adenine phosphoribosyltransferase) in transgenic (PCR +) and control non-transgenic (PCR-) TUB-SAMdc-Nt *Arabidopsis thaliana* plants.

**FIG. 14** (A) and (B) show hand-cut unfixed stem sections of 3 month-old *Nicotiana benthamiana* specimens, stained with the lignin-specific dye phloroglucinol in a control non-transgenic plant (A) and in a transgenic plant 4CL-SAMdc-Nt30 (B), which exhibits a high transgene expression level. (D) shows a homozygous transgenic plant in the T2 segregating population from the 4CL-SAMdc-Nt30 T1 transformant compared to a control non-transgenic sibling plant (C). Note the decrease in vessel element number and size in (B) and the general decrease in lignin staining in (D).

### DETAILED DESCRIPTION

The present invention relates to processes for genetic manipulation of lignin content and/or lignin composition in plants. Applications of the invention include, but are not limited to, the improvement of digestibility of forage crops through lignin reduction, the improvement of cellulose fibre extraction during papermaking through reduction of lignin in woody trees, and the improvement of lignin extraction during the pulping process through the increase of S/G (syringyl lignin/guaiacyl lignin) ratios in woody trees.

Industrial production of cellulose from woody trees requires the chemical removal of lignin from the wood extract during the pulping process, which makes use of large amounts of concentrated sodium hydroxide, sodium bissulfide and other hazardous substances. For high-quality paper production, residual lignin needs to be further removed by an additional bleaching step involving the use of chlorine oxides and other extremely hazardous substances (Petit-Conil, Ph.D thesis, Institut Nationale Polytechnique, Grenoble, France (1995)). The overall process is costly and represents an enormous environmental challenge. For this reason, reducing lignin content in woody plants, typically trees, is expected to lessen the chemical and energy demands of these highly expensive extraction processes and should also reduce the amount of effluent material, a major potential environmental pollutant that is both difficult and expensive to process. Moreover, increasing the syringyl/guaiacyl ratio of lignin in woody plants is expected to alter the solubility characteristics of lignin in the chemical extractants used favorably, also leading to reduced usage of hazardous, as well as expensive chemicals and lowered energy requirements (Huntley et al., Tree Biotechnology Symposium (2003)).

Plants that can be engineered in accordance with the invention include, those that contain lignin. Lignin-containing plants include, but are not limited to, trees such as Conifers such as loblolly pine *(Pinus taeda),* slash pine *(Pinus elliotii),* ponderosa pine *(Pinus ponderosa),* lodgepole pine *(Pinus contorta),* and Monterey pine *(Pinus radiata);* Douglas-fir *(Pseudotsuga menziesii);* Western hemlock *(Tsuga canadensis);* Sitka spruce *(Picea glauca);* redwood *(Sequoia sempervirens);* true firs such as silver fir *(Abies amabilis)* and balsam fir *(Abies balsamea);* and cedars such as Western red cedar *(Thuja plicata)* and Alaska yellow-cedar *(Chamaecyparis nootkatensis).*

Lignin-containing plants also include grasses such as maize, sorghum, rice, sugarcane and tall fescue. Also included are forage crops, such as alfalfa, lolium, festuca and clover. 33

It should be understood that the word "plant" is used broadly herein to include any lignin-containing plant material that can be genetically manipulated, including, but not being limited to, differentiated or undifferentiated plant cells, protoplasts, whole plants, plant tissues, or plant organs, or any component of a plant such as a leaf, stem, root, bud; tuber, fruit, rhizome, or the like.

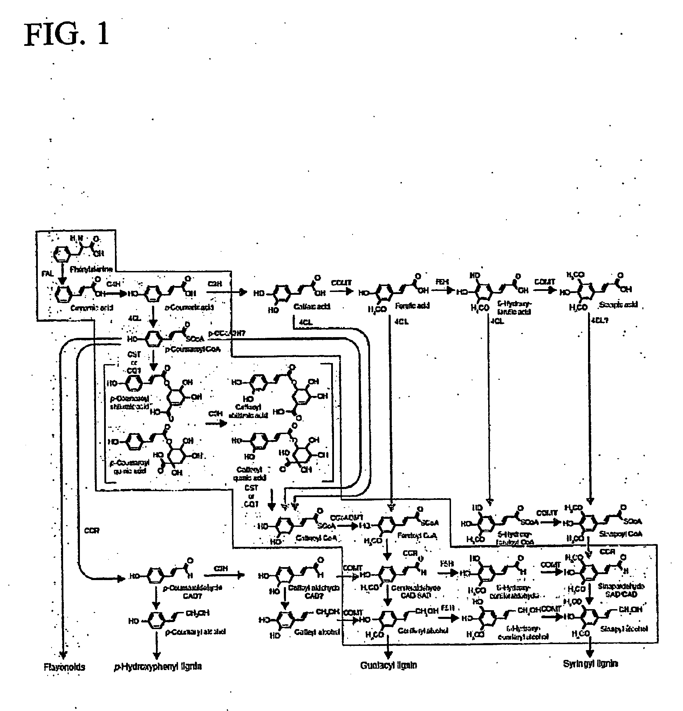

Lignin is synthesized via condensation of lignin monomeric units, known as monolignols, which are synthesized through the phenylpropanoid biosynthetic pathway (Boerjan et al., Annu. Rev. Plant Biol. 54: 519-546 (2003)) (FIG. 1). This pathway is also responsible for the production of a wide range of compounds including flavonoid pigments, isoflavonoids, coumarin phytoalexins and cell division-promoting dehydrodiconiferyl glucosides (Steeves et al., Phytochem. 57: 1085-1093 (2001)). Briefly, phenylalanine is deaminated to produce p-coumaric acid. p-coumaric acid is subsequently hydroxylated and methylated, to yield caffeic acid, ferulic acid, 5-hydroxyferulic acid and sinapic acid. By subsequent action on these acid intermediates by a hydroxycinnamate, i.e., CoA ligase, p-coumaroyl CoA, caffeoyl-CoA, feruloyl CoA, 5-hydroxyferuloyl CoA and sinapoyl CoA are formed. p-coumaroyl CoA, feruloyl CoA and sinapoyl CoA are eventually converted to hydroxyphonyl lignin, guaiacyl lignin and syringyl lignin, respectively (Boerjan et al., Annu. Rev. Plant Biol. 54: 519-546 (2003)). It should be noted that several steps in the monolignol biosynthesis pathway represent S-adenosylmethionine-dependent methylation reactions, evidencing the importance of this methionine-derived substrate in the lignin biosynthesis process, as will be discussed later.

The terms "reduced lignin content" or "decreased lignin content" used herein to describe a plant of refer to a quantitative reduction in the amount of lignin in the plant when compared to the amount of lignin in a wild-type plant. A quantitative reduction of lignin can be assayed by several methods, as for examples the Klason lignin assay (Kirk et al., Method in Enzymol., 161:87-101 (1988)) or acetyl bromide assay of lignin (Iiyama et al. Wood Sci. Technol., 22:271-280 1988)).

The lignin content in the engineered plant can be reduced to levels of about 5% to about 90%, preferably about 10% to about 75%, even more preferably about 15% to about 65% of the lignin content of the wild-type plant A plant of the disclosure may have a lignin content of about 20% to about 60% of the wild-type lignin content.

The term "altered lignin composition" as used herein to describe a plant refers to quantitative alteration in the relative amounts of syringyl and guaiacyl lignin units in the engineered plant compared to a wild-type plant. Preferably, a plant having altered syringyl and guaiacyl lignin composition exhibits a reduced guaiacyl lignin content compared to the guaiacyl lignin content of a wild-type plant; more preferably, it exhibits an increased pairwise syringyl lignin/guaiacyl lignin ratio, i.e., "S/G ratio," compared to the S/G ratio of a wild-type plant. Plants can be assayed to determine their S/G ratios in comparison to the ratio of a wild-type plant using several different assay methods, including those described by Rolando et al (Methods in Lignin Chemistry, Springer, New York (1992)). Plants can exhibit an overall decrease in lignin content when compared to a wild-type plant, while exhibiting an increased S/G ratio.

The term "transgenic" as used herein describes a plant that has been genetically transformed with a DNA construct comprising a suitable promoter sequence, preferably a cambium/xylem-preferred promoter like those disclosed in copending PCT patent application No. PCT/BR2005/000041, filed March 28, 2005 published as WO 2005/096805 operably linked to a gene encoding a SAMdc enzyme isoform. Alternatively, constitutive or other tissue-specific promoters can be operably linked to a gene encoding the enzyme SAMdc. The gene encoding the enzyme may also be used per se.

Transgenic plants of the disclosure are characterized by reduced lignin content. Reduced and/or altered lignin content in the genetically engineered plant is preferably achieved via reduction in the availability of SAM in the plant tissues wherein lignin deposition occurs. SAM is a substrate involved in methylation reactions in various biochemical steps of the lignin biosynthetic pathway.

Transgenic plants of the disclosure contain a DNA construct comprising a cambium/xylem-preferred promoter such as those described in the PCT patent application cited supra, operably linked to a gene encoding a SAMdc enzyme isoform, leading to increased activity in the plant's vascular system of a gene encoding SAMdc, a key enzyme in the conversion of SAM into polyamines. Over-expression of SAMdc is expected to result in an increased conversion of SAM into decarboxylated SAM (dSAM), therefore reducing the amount of SAM available to SAM-dependent methylation of intermediates in the monolignol biosynthesis pathway, consequently impairing lignin biosynthesis and deposition without affecting other plant functions.

The present invention utilizes nucleotide sequences corresponding to cDNAs or genomic sequences that encode SAMdc, disclosed herein as SEQ ID NOS: 1, 3, 5, 7, 9, 11, 13, 15, 17 and 19. The disclosure further provides a method for altering the lignin content in plant tissue, such as fiber cells of xylem, by transforming a plant with a SAMdc coding sequence which is expressed in a xylem-specific manner and causes increased conversion of SAM into dSAM, leading to decreased SAM availability for monolignol biosynthesis and consequent reduction in the vascular tissue's lignin content

According to a first aspect a method to modify the content and/or composition of lignin in plant tissues by controlling the activity of SAMdc is provided. The activity levels of SAMdc are increased, preferably in fibers of stem xylematic vascular tissues. Over-expression of SAMdc leads to enhanced conversion of SAM into polyamines, competing with the utilization of SAM in the monolignol biosynthesis pathway. The alteration in SAM levels specifically in the vascular tissues of a plant is expected to modify the plant's ability to synthesize and/or deposit lignin.

Genes encoding SAM-decarboxylase have been isolated. cDNA and genomic sequences have been characterized and may be used to modify the lignin content and/or the lignin composition of plant tisuues and/or organs.

The terms "encoding" and/or "coding" refer to the process by which a gene, through the mechanisms of transcription and translation, provides information to a cell from which a series of amino acids can be assembled into a specific amino acid sequence to produce an active enzyme. Because of the degeneracy of the genetic code, certain base charges in DNA sequence do not change the amino acid sequence of a protein. It is therefore understood that modifications in the DNA sequences encoding SAMdc which do not substantially affect the functional properties of SAMdc enzyme are contemplated.

The term "expression", as used herein, refers to the production of the protein product encoded by a gene. "Overexpression" refers to the production of a gene product in transgenic organisms that exceeds levels of production in normal or non-transformed organisms.

Nucleotide sequences encoding SAMdc enzyme are used by the invention. These sequences may be derived from cDNA, from genomic DNA or may be synthesized *ab initio.*

A cDNA clone encoding a SAMdc enzyme has been obtained from a tobacco (*Nicotiana tabacum*) cDNA pool. This cDNA clone is hereinafter called SAMdc-Nt, and set form herein as SEQ ID NO.: 1.

Two cDNA clones encoding SAMdc have been obtained from a *Populus deltoides* cDNA library. These are set forth herein as SEQ ID NOs.: 3 and 5, respectively:

DNA sequences encoding SAMdc may also be from genomic DNA fragments containing the entire gene. A genomic DNA fragment of a gene may differ from the corresponding cDNA in that introns may be present. The introns are not transcribed into mRNA (or, if so transcribed, are subsequently cut out).

Oligonucleotide primers have been used to isolate a SAMdc gene from *Populus deltoides* by PCR. One genomic clone is set forth herein as SEQ ID NO.: 7. The cDNA produced from this gene in a living cell corresponds to the cDNA set forth herein as SEQ ID NO.: 3. Moreover, a mutant form of the DNA sequence set forth herein under SEQ In NO.: 7 is disclosed herein as SEQ ID NO.: 19.

Also disclosed herein are SAMdc cDNA sequences obtained from *Arabidopsis thaliana* (SEQ ID NO.: 9), *Xanthomonas axonopodis* pv. citri (SEQ ID NO.: 11), *Datura stramonium* (SEQ ID NO.: 13) and *Oryza sativa* (SEQ ID NOs.: 15 and 17).

The aforementioned nucleotide sequences are disclosed herein. A SAMdc DNA sequence may be isolated as cDNA or genomic DNA from any suitable plant species using oligonucleotide primers or probes based on DNA or protein sequences disclosed herein. Specific examples of plant species from which SAMdc genes may be isolated include, but are not limited to, dicotyledons such as Cucurbitaceae, Solanaceae, Brassicaceae, Papilionaceae such as alfalfa and *Vigna unguiculata,* Malvaceae, Asteraceae, Malpighiaceae such as *Populus,* Myrtaceae such as *Eucalyptus,* or monocotyledons such as gramineae, including wheat, barley, and corn. DNA libraries from bacteria, such as *Xylella,* algae, fungi, yeast or animals may also be probed for SAMdc sequences in order to obtain SAMdc-coding sequences.

"SAMdc DNA sequence," as used herein, refers to any nucleic acid molecule with a nucleotide sequence capable of hybridizing under stringent conditions with any of the sequences disclosed herein, and coding for a polypeptide with SAMdc enzyme activity equivalent to the proteins having amino acid sequences disclosed herein under SEQ ID NOS: 2, 4, 6, 8, 10, 12, 14, 16, 18. The term also includes sequences which cross-hybridize with SEQ ID NO:1, SEQ ID NO:3, SEQ ID NO:5, SEQ ID NO:7, SEQ ID NO:9, SEQ ID NO:11, SEQ ID NO:13, SEQ ID NO:15, or SEQ ID NO:17, preferably having at least 65% homology or identity with one or more of SEQ ID NO: 1,3,5,7,9,11,13,15, 17 or 19. The nucleotide sequences may encode a protein which is homologous to the predicted gene product disclosed herein under any of SEQ ID NO: 2, SEQ ID NO:4, SEQ ID NO:6, SEQ ID NO:8, SEQ ID NO:10, SEQ ID NO:12, SEQ ID NO:14, SEQ ID NO:16, or SEQ ID NO:18.

Further useful sequences include any nucleic acid molecules comprising any of the above base sequences with one or more bases deleted, substituted, inserted, or added, and coding for a polypeptide with SAMdc enzyme activity equivalent to those sequences or a protein which is homologous to the protein encoded by SEQ ID NO:1, SEQ ID NO:3, SEQ ID NO:5, SEQ ID NO:7, SEQ ID NO:9, SEQ ID NO:11, SEQ ID NO:13, or by SEQ ID NO:15; or by SEQ ID NO:17. "Stringent conditions", as referred to here, means conditions under which only base sequences coding for a polypeptide with SAMdc enzyme activity equivalent to the SAMdc enzyme encoded by a specific SAMdc gene sequence form hybrids with the specific SAMdc sequence (referred to as specific hybrids), and base sequences coding for polypeptides with no such equivalent activity do not form hybrids with the specific sequence (referred to as non-specific hybrids). One with ordinary skill in the art can readily select such conditions by varying the temperature during the hybridization reaction and washing process, the salt concentration during the hybridization reaction and washing process, and so forth. Specific examples include, but are not limited to, conditions under which hybridization is brought about in 3.5xSSC, 1xDenhardt's solution, 25mM sodium phosphate buffer (pH 7.0), 0.5% SDS, and 2mM EDTA for 18 hours at 65°C, followed by 4 washes of the filter at 65°C for 20 minutes, in 2xSSC, 0.1% SDS, and a final wash for up to 20 minutes in 0.5xSSC, 0.1% SDS, or 0.3xSSC and 0.1% SDS for greater stringency, and 0.1xSSC, 0.1% SDS for even greater stringency. Other conditions may be substituted, as long as the degree of stringency in equal to that provided herein, using a 0.5xSSC final wash.

The "base sequences with one or more bases deleted, substituted, inserted, or added" referred to here are widely known by those having ordinary skill in the art to retain physiological activity even when the amino acid sequence of a protein generally having that physiological activity has one or more amino acids substituted, deleted, inserted, or added. Nucleotide sequences that have such modifications and that code for a SAMdc enzyme isoform are included. For example, the poly A tail or 5' of 3' end nontranslation regions may be deleted, and bases may be deleted to the extent that amino acids are deleted. Bases may also be substituted, as long as no frame shift results. Bases may also be "added" to the extent that amino acids are added. However, it is essential that such modifications do not result in the loss of SAMdc enzyme activity. Such modified DNA can be obtained by modifying the DNA base sequences so that amino acids at specific sites are substituted, deleted, inserted, or added by site-specific mutagenesis, for example (Nucleic Acid Research, Vol. 10, No. 20, 6487-65 00 (1982)).

A further way of obtaining a SAMdc DNA sequence is to synthesise it *ab initio* from the appropriate bases, for example, by using the appropriate cDNA sequence as a guide.

Some or all of the SAMdc sequences may be incorporated into DNA constructs suitable for plant transformation. These DNA constructs may then be used to modify SAMdc gene expression in plants.

According to a further aspect there are provided DNA constructs comprising any one of the SAMdc DNA sequences disclosed herein under the control of a transcriptional initiation region operative in plants, so that the construct can generate RNA in plant cells. Preferably, the transcriptional initiation region is part of an organ or tissue-specific plant promoter, such as any of those referred to, supra. More preferably, the tissue-specific promoter, when operably linked to the SAMdc DNA sequence, ensures transcription in specific cell types, tissues or organs such that the lignin metabolism can be specifically targeted without affecting other plant functions.

The lignin content and/or composition and related characteristics of plant parts may be modified by transformation with a DNA construct The disclosure also provides plant cells containing such constructs; plants derived therefrom having modified SAM-decarboxylase gene expression; and seeds of such plants.

DNA constructs may comprise a base sequence of a minimum length to generate a mRNA and consequently a polypeptide retaining SAMdc enzymatic activity. For convenience, it will generally be found suitable to use sequences between about 100 and about 1000 bases in length but there is no theoretical upper limit to the base sequence length. The preparation of such constructs is described in more detail below.

As a source of the DNA base sequence for transcription, a suitable cDNA or genomic DNA or synthetic polynucleotide may be used. Methods for the isolation of suitable SAM-decarboxylase sequences are described, supra. Sequences coding for the whole, or substantially the whole, of the enzyme may thus be obtained. Suitable lengths of this DNA sequence may be cut out for use by means of restriction enzymes. When using genomic DNA as the source of a partial base sequence for transcription, it is possible to use either intron or exon regions or a combination of both.

To obtain constructs suitable for modifying expression of SAMdc in plant cells, the cDNA sequence as found in the enzyme cDNA or the gene sequence as found in the chromosome of the plant may be used. Recombinant DNA constructs may be made using standard techniques. For example, the DNA sequence for transcription may be obtained by treating a vector containing said sequence with restriction enzymes to cut out the appropriate segment. The DNA sequence for transcription may also be generated by annealing and ligating synthetic oligonucleotides or by using synthetic oligonucleotides in a polymerase chain reaction (PCR) to give suitable restriction sites at each end. The DNA sequence is then cloned into a vector containing upstream promoter and downstream terminator sequences.

For example, constructs may be made as follows. A suitable vector containing the desired base sequence for transcription (such as the SAMdc-Nt cDNA clone) is treated with restriction enzymes to cut the sequence out. The DNA strand so obtained is cloned into a second vector containing the desired promoter sequence and the desired terminator sequence. In a DNA construct used by the invention, the transcriptional initiation region may be derived from any promoter operative in plants, so that the construct can generate RNA in plant cells. Preferably, the transcriptional initiation region is part of an organ or tissue-specific plant promoter such as a cambium/xylem-preferred promoter as described, supra. More preferably, the tissue-specific promoter, when operably linked to the SAMdc DNA sequence, ensures transcription in specific cell types, tissues or organs such that the lignin metabolism can be specifically targeted without affecting other plant functions. Suitable terminator sequences may be that from the *Agrobacterium tumefaciens* nopaline synthase gene (the *Nos* 3' end).

The transcriptional initiation region (or promoter) operative in plants may be such that it drives expression in certain tissues, organs or cell types, leading to the overexpression of SAMdc in those tissues and affecting lignin content and/or composition without compromising other biological functions. For example, by using vascular system-specific, xylem-specific or xylem-preferred promoters one can modify SAMdc activity specifically in lignin-synthesizing tissues such as vascular tissues, especially xylem. The use of a constitutive promoter in general affects enzyme levels and functions in all parts of the plant, while use of a tissue-preferred promoter permits targeting of the modified gene expression to specific plant parts, leading to more controllable phenotypes. Thus, in using the invention, it may be found convenient to use a promoter that will give expression during xylem development and/or xylem lignification, whereby the SAMdc enzyme would only be overproduced in the organ(s) or tissue(s) or cell type(s) in which its action is required. Vascular tissue-specific, xylem-specific promoters, vascular tissue-preferred and xylem-preferred promoters that could be used include, but are not limited to, the xylem-preferred tubulin (TUB) gene promoter, the xylem-preferred lipid transfer protein (LTP) gene promoter and the xylem-preferred coumarate-4-hydroxylase (C4H) gene promoter, as described, supra.

Constructs may be used to transform any plant using any suitable transformation technique. Both monocotyledonous and dicotyledonous angiosperm or gymnosperm plant cells may be transformed in various ways known to the art (Klein et al., Biotechnology, 4: 583-590 (1993); Bechtold et al., C. R. Acad. Sci. Paris 316:1194-1199 (1993); Bent et al., Mol. Gen. Genet. 204:383-396 (1986); Paszowski et al., EMBO J. 3: 2717-2722 (1984); Sagi et al., Plant Cell Rep. 13: 262-266 (1994)).

Examples of genetically modified plants according to the present disclosure include all woody trees, including, but not limited to, Conifers as, for example, loblolly pine (*Pinus taeda*)*,* slash pine (*Pinus elliotii*), ponderosa pine (*Pinus ponderosa*)*,* lodgepole pine (*Pinus contorta*)*,* and Monterey pine (*Pinus radiata*); Douglas-fir (*Pseudotsuga menziesii*); Western hemlock (*Tsuga canadensis*); Sitka spruce (*Picea glauca*); redwood (*Sequoia sempervirens*); true firs such as silver fir (*Abies amabilis*) and balsam fir (*Abies balsamea*); and cedars such as Western red cedar (*Thuja plicata*) and Alaska yellow-cedar (*Chamaecyparis nootkatensis*). Other plants that may be modified by the process of the invention .

Use of SAMdc constructs allows modification of SAMdc enzyme activity and thus provides a method for modification of lignin content and/or composition. It is also possible to modify SAMdc activity while also modifying the activity of one or more additional enzymes. For example, the additional enzymes may be involved in cell wall metabolism, cellulose biosynthesis or lignin metabolism. Cell wall-related enzymes that may be modified in combination with SAM-decarboxylase include, but are not limited to, cellulose synthase, sucrose synthase, expansin and tubulin. Other proteins involved in cell wall metabolism and biochemical properties that may be modified in combination with SAMdc include xylem- or cell wall-related transcription factors.

### EXAMPLES

### General Methods

All technical terms used herein are terms commonly used in biochemistry, molecular biology and agriculture, and can be understood by one of ordinary skill in the art to which this invention belongs. Those technical terms can be found in: Molecular Cloning: A Laboratory Manual, 3rd ed., vol. 1-3, ed. Sambrook and Russel, Cold Spring Harbor Laboratory Press, Cold Spring Harbor, N.Y., 2001; Current Protocols in Molecular Biology, ed. Ausubel et al., Greene Publishing Associates and Wiley-Interscience, New York, 1988 (with periodic updates); Short Protocols in Molecular Biology: A Compendium of Methods from Current Protocols in Molecular Biology, 5th ed., vol. 1-2, ed. Ausubel et al., John Wiley & Sons, Inc., 2002; Genome Analysis: A Laboratory Manual, vol. 1-2, ed. Green et al., Cold Spring Harbor Laboratory Press, Cold Spring Harbor, N.Y., 1997. Methods involving plant biology techniques are described herein and are described in detail in methodology treatises such as Methods in Plant Molecular Biology: A Laboratory Course Manual, ed. Maliga et aL, Cold Spring Harbor Laboratory Press, Cold Spring Harbor, N.Y., 1995. Various techniques using PCR are described, e.g., in Innis et al., PCR Protocols: A Guide to Methods and Applications, Academic Press, San Diego, 1990 and in Dieffenbach and Dveksler, PCR Primer A Laboratory Manual, 2nd ed., Cold Spring Harbor Laboratory Press, Cold Spring Harbor, N.Y., 2003. PCR-primer pairs can be derived from known sequences by known techniques such as using computer programs intended for that purpose (e.g., Primer, Version 0.5, 1991, Whitehead Institute for Biomedical Research, Cambridge, MA). Methods for chemical synthesis of nucleic acids are discussed, for example, in Beaucage and Caruthers (1981) Tetra. Letts. 22:1859-1862 and Matteucci and Caruthers (1981) J. Am. Chem. Soc. 103:3185.

Restriction enzyme digestions, phosphorylations, ligations and transformations were done as described in Sambrook et al., Molecular Cloning: A Laboratory Manual, Second Edition (1989) Cold Spring Harbor Laboratory Press. All reagents and materials used for the growth and maintenance of bacterial cells were obtained from Aldrich Chemicals (Milwaukee, Wis.), DIFCO Laboratories (Detroit, Mich.), Invitrogen (Gaithersburg, Md.), or Sigma Chemical Company (St. Louis, Mo.) unless otherwise specified.

Specific examples of methods for obtaining S-adenosylmethionine decarboxylase enzyme genes as well as methods for introducing the target gene using *Agrobacterium* to produce plant transformants are given below.

### EXAMPLE 1

### Isolation of the tobacco cDNA encoding SAMdc enzyme

### (a) Preparation of mRNA from tobacco leaves and cDNA synthesis

RNA was extracted from leaf tissue of *Nicotiana tabacum* using Trizol reagent (Invitrogen). A cDNA pool was prepared from the isolated total RNA using a commercially available cDNA Superscript II Amplification Kit (Invitrogen) or the like. The cDNA pool can then be used in RT-PCR experiments in which the isolated total RNA is used as template, and Superscript II reverse transcriptase (Invitrogen) and oligo(dT) primer are used to synthesize the first-strand cDNA and double-stranded cDNA is obtained by the subsequent polymerase reaction, using gene-specific primers.

### (b) Design of PCR Primers

A DNA sequence coding for SAMdc from *Nicotiana tabacum* has already been determined and deposited in the GenBank under accession number AF033100. Based on this sequence, DNA oligomers were synthesized as primers for PCR, including either the region around the first codon ATG or around the termination codon of the main ORF encoding the SAMdc enzyme.

Primers were designed to amplify the entire coding region of the SAMdc main ORF, i.e., from the ATG through the translation stop codon. The sequences of the primers are given below for the tobacco SAMdc gene:

| | |
|---|---|
| **SAMdc_Nt1** Length: 24 | SEQ ID NO:20 |
| ATCCCATGGATTCGGCCTTGCCTG | |
| **SAMdc_Nt2** Length: 34 | SEQ ID NO:21 |
| GTCTAGACTACTCCTTCTCTTCTTCTCTTCATC | |

### (c) PCR amplification of SAMdc from Nicotiana tabacum

The cDNA pool obtained in (a) was used as template, and the primers designed in (b) were used for PCR. The PCR steps involved 40 cycles of 1 minute at 94°C., 1 minute at 52°C., and 2 minutes at 72°C followed by an extra step of elongation at 72°C for 7 minutes. The PCR products were isolated by gel electrophoresis on 1.0% agarose followed by ethidium bromide staining of the electrophoresed gel and detection of amplified bands on a UV transilluminator. The detected amplified band was verified and cut out of the agarose gel with a razor. The pieces of gel were transferred to 1.5 mL microtubes, and the DNA fragments were isolated and purified using a GFX PCR clean-up and gel band purification kit (Amersham). The recovered DNA fragments were subcloned to the pGEM-T cloning vector (Promega), transformed into *E. coli,* and then used to prepare plasmid DNA. in the usual manner, which was then sequenced by the dideoxy method (Messing, Methods in Enzymol., 101, 20-78 (1983)) using the BigDye chemistry (Applied Biosystems), yielding the DNA sequence disclosed herein under SEQ ID NO. 1 for use according to embodiments described in this patent specification.

### EXAMPLE 2

### Preparation of Transgenic Arabidopsis and Nicotiana Plants

The gene obtained in Example 1 above was introduced into a plant host to produce transgenic *Arabidopsis* and *Nicotiana* plants.

### (1) Preparation of constructs and transformation of Agrobacterium

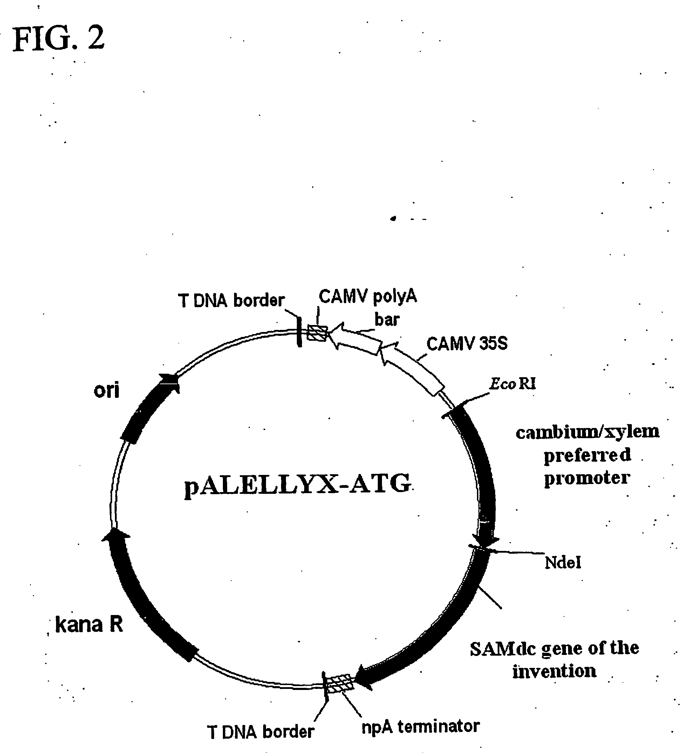

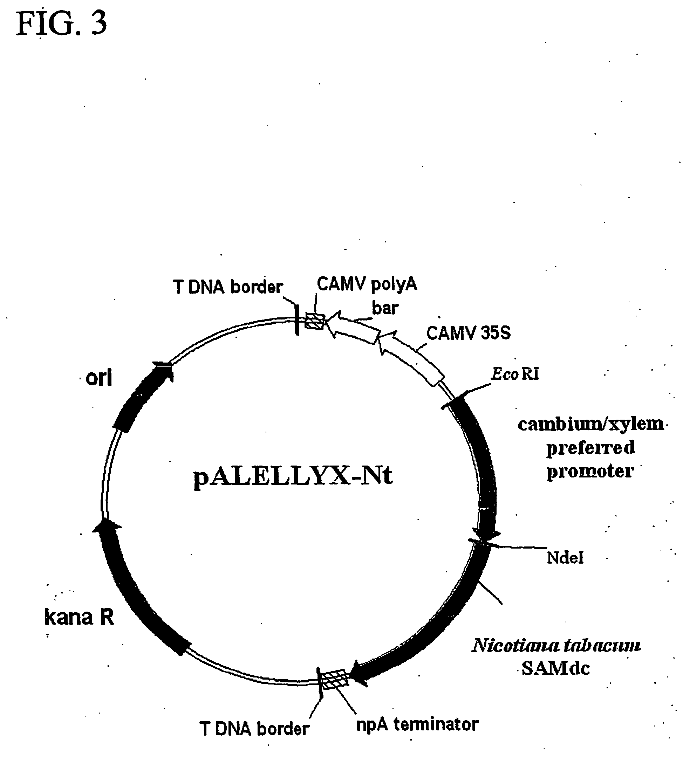

Expression constructs can be prepared by cleaving the SAMdc genes obtained in 1 above with suitable restriction enzymes so as to include all of the open reading frame and inserting the gene into the plant transformation vector pALELLYX-ATG (FIG. 2) together with an appropriate promoter. For example, the tobacco SAMdc gene obtained in Example 1 was cloned into the aforementioned expression vector downstream to a xylem-preferred tubulin gene (TUB) promoter from *Populus deltoides* as set forth in PCT patent application No. PCT/BR2005/000041, filed March 28, 2005, published as WO2005/096805 wich claims for the priority date of Serial No. 60/560,227, filed April 6, 2004 (FIG. 3). The resulting expression construct is amplified in *E. coli,* and then transformed by tripartite conjugation (Nucleic Acid Research, 12, 8711 (1984)), freeze thawing, electroporation, chemical transformation or the like into *Agrobacterium tumefaciens* C58, GV3101 or the like.

### (2) Agrobacterium-mediated transformation of Arabidopsis thaliana

*Arabidopsis thaliana* Columbia plants were transformed using an *Agrobacterium tumefaciens* mediated transformation protocol (Bechtold et al., (1993) C. R. Acad. Sci. Paris 316:1194-1199; Bent et al., (1986) Mol. Gen. Genet. 204:383-396) with the construct containing the tobacco SAMdc gene obtained in Example 1 operably linked to the promoter of a xylem preferred gene (TUB). The construct also contains the selectable marker gene *Bar* that confers resistance to herbicidal phosphinothricin analogs like ammonium gluphosinate (Thompson et al. (1987) EMBO J. 9:2519-2523).

Seeds of *Arabidopsis thaliana* ecotype Columbia were sown in pots containing vermiculite. Plants were grown at 16/8 hours dark/light regime at 22°C. After 4-5 weeks plants were transformed with the *Agrobacterium tumefaciens* strain GV3101 (C58C1 rifampicin) pMP90 (gentamicin) (Bent et al., (1986) Mol. Gen. Genet. 204:383-396) harboring the plasmid vector comprising the SAMdc gene of interest operably linked to the TUB promoter.

For plant transformation, 1 liter of LB medium containing rifampicin, gentamycin and kanamicin was inoculated with an aliquot of overnight starter *Agrobacterium* culture. The culture was grown overnight at 28°C in a rotatory shaker, until OD600 is ≥0.8. The *Agrobacterium* was precipitated by centrifugation and the bacterial pellet was resuspended in ∼300 ml of 5% sucrose and 0.03% Silwet L-77 (Witco). This *Agrobacterium* suspension was sprayed onto the plants. The pots were then placed in a tray which is covered with plastic wrap to maintain humidity. The plants were grown at 16/8 hours dark/light regime at 22°C through maturity to set seeds.

Seeds were harvested, surface-sterilized in a solution containing 50% bleach and 0.02% Triton X-100 for 7 minutes. Seeds were then rinsed 3 times in sterile distilled water and plated out in MS medium containing 6 mg/l of Finale (Bayer) as selective agent. After 5 to 7 days, transformants were visible as green plants. Transformed plants were transferred onto new selection plates and after 6-10 days were transferred to pots containing vermiculite and grown under conditions of 16 hours light/8 hours dark at 22°C. After three weeks, the inflorescence stems were cut close to their bases twice a week for a period of one month in order to induce secondary growth at the base of the rosette before the plants were analyzed.

### (3) Agrobacterium-mediated transformation of Nicotiana benthamiana

Transformation of *Nicotiana benthamiana* was accomplished using the leaf disk method of Horsch et al. (Science 227, 1229, (1985)), using a contruct comprising the tobacco SAMdc gene obtained in Example 1 operably linked to the promoter of a xylem-preferred gene (4CL; Hu et al. (1998) PNAS 95:5407-5412). The transformants were selected by growing on Murashige and Skoog medium (Sigma, St. Louis, Mo.) containing 100 milligrams/liter of BASTA herbicide and 500 mg/L carbenicillin (Sigma). The transformed tobacco shoots are allowed to root on the Murashige and Skoog medium, and are subsequently transferred to soil and grown in the greenhouse.

### (4) PCR verification of foreign gene insertion into the host plant genome

PCR can be used to verify the integration of the gene construct in the genome of transgenic plants. Two specific primers are synthesized for the construct and used to ECR-amplify the corresponding construct from genomic DNA of *Arabidopsis* or *Nicotiana* transformants. For the TUB-SAMdc-Nt construct, which contains the tobacco SAMdc main ORF under the control of the *Populus* xylem-preferred tubulin gene promoter, two specific primers were synthesized that amplify a 1.8kb fragment:

| | |
|---|---|
| **Tub_check1** Length: 25 | SEQ ID NO:22 |
| TATCGTTTTACTTCACTGGTCGGTG | |
| **SAMdc_Nt2** Length: 34 | SEQ ID NO:21 |
| GTCTAGACTACTCCTTCTCTTCTTTCTCTTCATC | |

For the 4CL-SAMdc-Nt construct, which contains the tobacco SAMdc main ORF under the control of the *Populus* xylem-preferred 4CL gene promoter, two specific primers were synthesized that amplify a 1.6 kb fragment:

| | |
|---|---|
| **4CL_seq** Length: 20 | SEQ ID NO:23 |
| AATCTCACCAACCCAACTCC | |
| **SAMdc_Nt2** Length: 34 | SEQ ID NO:21 |
| GTCTAGACTACTCCTTCTCTTCTTTCTCZTCATC | |

The PCR reaction mixture contained 100 ng genomic DNA of transformed plant, and 0.2 µM of each primer, 100 µM of each deoxyribonucleotide triphosphate, 1×PCR buffer and 2.5 Units of AmpliTaq DNA polymerase (Applied Biosystems) in a total volume of 50 µL. The cycling parameters were as follows: 94°C. for 1 minute, 50°C. for 1 minute and 72°C for 3 minutes, for 40 cycles, with 5 minutes at 72°C. extension. The PCR products were electrophoresized on an 1% agarose gel.

### (5) Determination of transgene expression level in transgenic plants

Semi-quantitative RT-PCR was used to detect the accumulation of tobacco SAMdc transcripts in stem tissue of the transgenic plants. Total RNA was isolated from 5-cm inflorescence stem cuts from transgenic *Arabidopsis* T1 plants (primary transformants) using Trizol reagent (GibcoBRL, USA) according to the manufacturer's instructions or from stem cuts of 3-months old transgenic *Nicotiana benthamiana* T1 plants using the CTAB method (Aldrich and Cullis(1993) Plant Mol. Biol. Report. 11:128-141).

cDNA was synthesized from 500 ng of total RNA using Superscript II RNase H- RT (Invitrogen, USA). Primers for the constitutive gene encoding adenine phosphoribosyltransferase (APRT) (Moffatt, B.A., McWhinnie, E.A., Agarwalb, S.K. and Schaff, D.A. (1994). Gene 143, I211-I216) were used as an internal control to normalize the quantity of total RNA used in each sample. The PCR was done with a 12.5-fold dilution of the first-strand cDNA under the following conditions: 94°C for 3 min and 27 cycles of 94°C for 1 min, 52 to 60°C for 45 s, and 72°C for 1 min and 30 s.

### EXAMPLE 3

### Histochemical Analysis of Transgenic Plants

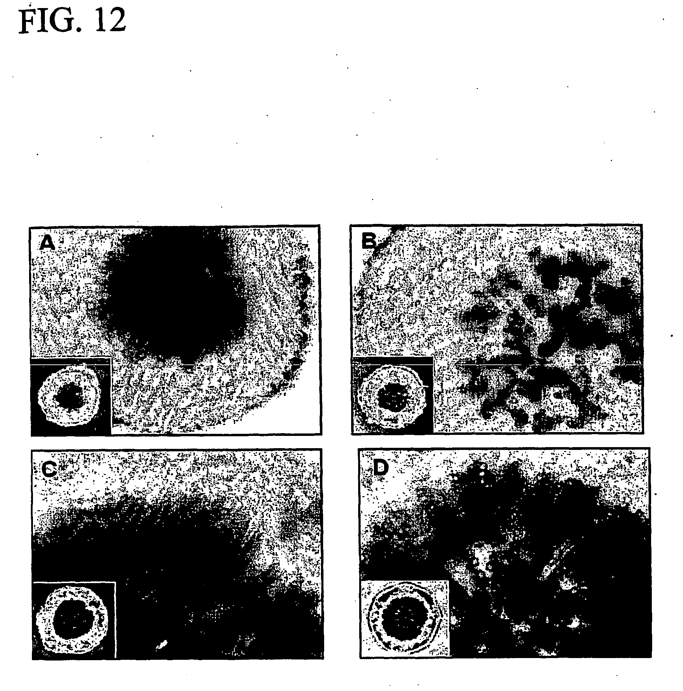

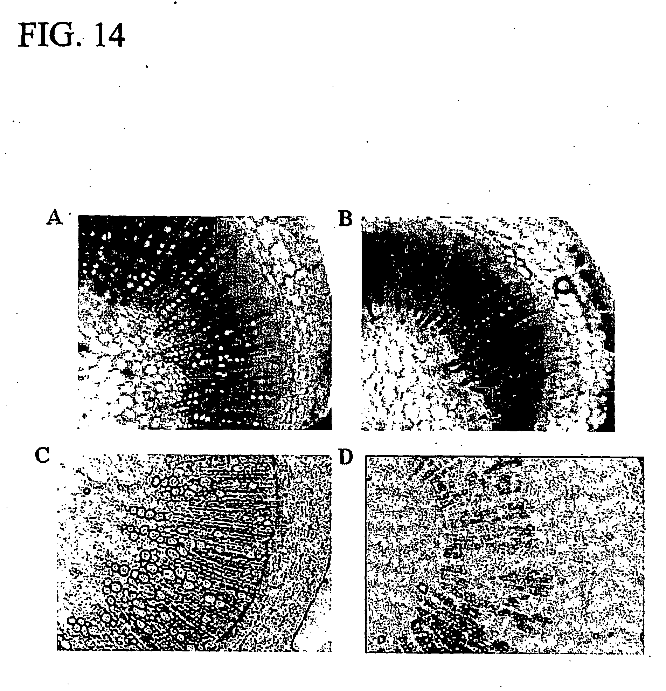

Histological staining of lignin can be performed to analyze the amount of lignin in the vascular system of transformed and control plants. Briefly, stems of *Arabidopsis* TUB-SAMdc-Nt transgenic, *Nicotiana benthamiana* 4CL-SAMdc-Nt and control non-transgenic plants were free-hand sectioned with a razor blade, and the resulting sections were stained for total lignin with phlomglucinol-HCl (1% phloroglucinol in 6N HCl) for 30 minutes (Zhong et al., Plant Physiol. 123: 59-69 (2000)). Alternatively, tissues were sectioned in a microtome (Leica RM2255) and subsequently stained with phloroglucinol. The histologically stained sections are observed under a dissection microscope using bright- and dark-field illumination (FIGs. 12 and 14).

### EXAMPLE 4

### Reduction in Lignin Content in Transgenic Plants Over-expressing SAMdc in the Vascular Tissue

The effect of over-expression of SAMdc in lignin biosynthesis in plant species was investigated in *Arabidopsis* and *Nicotiana.* It was found that over-expressing SAMdc in a xylem-preferred manner in transgenic *Arabidopsis* plants resulted in a reduction in lignin content (FIG. 12). Similar findings were obtained in two generations of transformed *Nicotiana* plants (FIG. 14). This finding indicates that over-expression of SAMdc is an efficient means for genetically engineering trees with low lignin content.

*Arabidopsis* transgenic plants were made following the methods described in Example 2. The first generation of transformants (T1) was analyzed using the methods described in Example 3 to assay lignin content, as well as vessel structure and vascular tissue anatomy. Several independent transformants were produced that contain a DNA expression construct comprising the *Nicotiana tabacum* SAMdc main ORF under the control of the *Populus* xylem-preferred tubulin gene promoter described in PCT patent application No. PCT/BR2005/000041, filed March 28, 2005, published as W0 2005/096805 which claims for the priority date of Serial No. 60/560,227 filed April 6, 2004, suppra.

SAMdc gene expression levels were measured in twenty independent T1 plants, using the methods set forth in Example 2. Stems of transgenic plants were used for RNA extraction followed by a semi-quantitative RT-PCR. SAMdc gene expression levels in transgenic plants were expressed as a percentage of the maximum expression level observed.

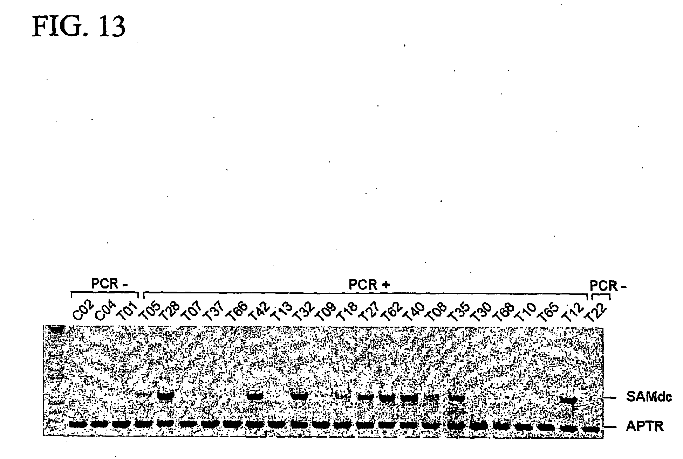

As shown in FIG. 13, the transgenic *Arabidopsis* plant TUB-SAMdc-Nt12 showed a high trasngene expression level (95% of the maxium level observerd), which was accompanied by a decrease in phloroglucinol pinkish red staining intensity (FIG. 12B) compared to a comparable control non-transformed plant (FIG. 12A), reflecting a reduction in lignin content, which provides evidence that enhanced SAMdc expression in specific vascular cell types leads to a reduction in the availability of the methyl group donor SAM and therefore impairs lignin monomer biosynthesis during xylem lignification. Notably, although it exhibits a marked reduction in lignin content compared to a wild-type plant, the *Arabidopsis* transformant TUB-SAMdc-Nt12 has been grown normally under' standard conditions, with no visible abnormal growth or morphology being observed.

Other T1 plants with low or non-existent expression of the SAMdc transgene, like TUB-SAMdc-Nt09, failed to show any difference in phloroglucinol staining compared to comparable control non-transformed plants (FIGs. 12C, D).

Similar results were obtained when the construct comprising the *Nicotiana tabacum* SAMdc main ORF under the control of the *Populus* xylem-preferred 4CL gene promoter (Hu et al. (1998) PNAS 95:5407-5412) was transformed into *Nicotiana benthamiana* plants. *Nicotiana benthamiana* transgenic plants were made following the methods described in Example 2. Several independent first-generation transformants (T1) were analyzed using the methods described in Example 3 to assay lignin content, as well as vascular tissue anatomy. As shown in FIG. 14, the transgenic *Nicotiana benthamiana* plant 4CL-SAMdc-Nt30 showed a marked decrease in the number of vessel elements in the vascular tissue (FIG. 14B) compared to a control non-transformed plant (FIG. 14A). This might reflect a reduction in the plant's ability to deposit lignin in the cell wall. Seeds from this transgenic plant were germinated to obtain a segregating population of plants in the following generation (T2). These segregants could be separated into three groups according to transgene expression level and plant size. Heterozygous and homozygous T2 plants possessed a reduction in size and a strong reduction in lignin content as assessed by phloroglucinol staining according to Example 3 (FIG. 14D), as compared to non-transgenic sibling plants (FIG. 14C), providing strong evidence that enhanced SAMdc expression in specific vascular cell types leads to a reduction in the availability of the methyl group donor SAM and therefore impairs lignin monomer biosynthesis during xylem lignification.

### SEQUENCE LISTING

<110> PAPES, Fabio
   ARRUDA, Paulo
<120> Polynucleotides, DNA Constructs and Methods for the Alteration of Plant Lignin Content
   and/or Composition
<130> ALEL 203.1 PCT
<140>
<141>
<150> US 60/602,440
<151> 2004-08-18
<160> 23
<210> 1
   <211> 1658
   <212> DNA
   <213> Nicotiana tabacum
   <220>
   <400> SEQ ID NO: 1 
atggagtcga aaggtggtaa aaactctagt agtaaatcct taccctacga agcacccctc ggctacagta 70

ttgaagacgt tcggccaaac ggtggaatca agaagttcag atcagctgct tactccaact gcgctcgcaa 140

accatcctga cattccttaa gcttctctcc tgcacgtgtc tcctgacaca aaaaagaaaa aatccccaaa 210

aaaagttcct tctgtcaatt gtttttgttg ttaaaccctc actccttttc ctcaatttct tccttctgct 280

gctttctgct cttgctctcc ttggctgtga acaatttctt ttaaaagatc atttgttgct gtgaacatat 350

ttttttttat ctaatggatt cggccttgcc tgtctctgcc attggttttg aaggtttcga gaagaggctt 420

gaaatttctt ttttcgagcc tggtctgttt gctgatccca acggaaaagg acttcgatct ctctcaaagg 490

cacaattgga tgagattctc ggacctgctg agtgcaccat agttgattcc ctatcaaatg acgatgttga 560

ttcttatgtc ctctccgagt cgagcctctt tgtttattct tacaagataa tcatcaaaac ctgtggcacc 630

acaaagttgc ttctcgcaat tccgcccatc ctaaagttgg ctgagaccct gtctctcaaa gtacaagacg 700

tgaggtatac ccgtgggagc ttcatttccc ctggcgctca gtcgtttcct catcgtcact tctctgaaga 770

agttgctgtc ctcgatggct attttggaaa gcttgctgcc ggtagcaagg ctgtgattat gggcagtcct 840

gacaaagcac agaaatggca tgtttactct gcctctgcag gacctattca gtctaatgac cctgtttaca 910

ctcttgagat gtgtatgact ggtttggaca gggagaaggc atctgtcttt tacaagactg aaggaagctc 980

ggctgctcat atgactgttc gatctggaat aaggaagatc ctccccaatt ctgagatatg cgattttgag 1050

tttgaaccct gtggttattc catgaattca attgaaggag ctgcactctc aaccattcac attaccccgg 1120

aagatggctt tagctatgct agctttgaag cagttgggta tgacatgaaa accatgaagc tgggtcccct 1190

ggttgagagg gtgctggcat gtttccagcc agatgagttc tctattgctt tgcatgctga tgttgctacc 1260

aagttactgg agcgtgtttg ctctcttgat gtgaaaggct actctcttgc tgagtggagt ccagaagaat 1330

ttggcaaggg tggttccatt gtctaccaga agttcaccag aactcctttc tgtggatctc ccaagtccgt 1400

tctgaagggc tgctggaaag aagatgaaga gaaagaagag aaggagtagt gtcttgaggg ctgtgttgtt 1470
 
ttttgtttca gtgtccgtgt ctgtctctgt ctgtgtcggt gtcgtttgtt ttttcagtgt ttctccgaat 1540

aaagtacttg atgtccaagc tgtgtcgttt ggatttgtaa tgccgatgtg caaattctga actattcttg 1610

gcttttgtg ttccacccga agccctatga acctgcattt tgaataaa 1658
<210> 2
   <211> 361
   <212> PRT
   <213> Nicotiana tabacum
   <400> SEQ ID NO: 2 
Met Asp Ser Ala Leu Pro Val Ser Ala Ile Gly Phe Glu Gly Phe Glu Lys Arg
1               5                   10                  15

Leu Glu Ile Ser Phe Phe Glu Pro Gly Leu Phe Ala Asp Pro Asn Gly Lys Gly
    20                  25                  30                  35

Leu Arg Ser Leu Ser Lys Ala Gln Leu Asp Glu Ile Leu Gly Pro Ala Glu Cys
            40                  45                  50

Thr Ile Val Asp Ser Leu Ser Asn Asp Asp Val Asp Ser Tyr Val Leu Ser Glu
55                  60                  65                  70

Ser Ser Leu Phe Val Tyr Ser Tyr Lys Ile Ile Ile Lys Thr Cys Gly Thr Thr
        75                  80                  85                  90

Lys Leu Leu Leu Ala Ile Pro Pro Ile Leu Lys Leu Ala Glu Thr Leu Ser Leu
                95                  100                 105

Lys Val Gln Asp Val Arg Tyr Thr Arg Gly Ser Phe Ile Phe Pro Gly Ala Gln
    110                 115                 120                 125

Ser Phe Pro His Arg His Phe Ser Glu Glu Val Ala Val Leu Asp Gly Tyr Phe
            130                 135                 140

Gly Lys Leu Ala Ala Gly Ser Lys Ala Val Ile Met Gly Ser Pro Asp Lys Ala
145                 150                 155                 160

Gln Lys Trp His Val Tyr Ser Ala Ser Ala Gly Pro Ile Gln Ser Asn Asp Pro
        165                 170                 175                 180

Val Tyr Thr Leu Glu Met Cys Met Thr Gly Leu Asp Arg Glu Lys Ala Ser Val
                185                 190                 195

Phe Tyr Lys Thr Glu Gly Ser Ser Ala Ala His Met Thr Val Arg Ser Gly Ile
    200                 205                 210                 215

Arg Lys Ile Leu Pro Asn Ser Glu Ile Cys Asp Phe Glu Phe Glu Pro Cys Gly
            220                 225                 230

Tyr Ser Met Asn Ser Ile Glu Gly Ala Ala Leu Ser Thr Ile His Ile Thr Pro
235                 240                 245                 250

Glu Asp Gly Phe Ser Tyr Ala Ser Phe Glu Ala Val Gly Tyr Asp Met Lys Thr
        255                 260                 265                 270

Met Lys Leu Gly Pro Leu Val Glu Arg Val Leu Ala Cys Phe Gln Pro Asp Glu
                275                 280                 285

Phe Ser Ile Ala Leu His Ala Asp Val Ala Thr Lys Leu Leu Glu Arg Val Cys
    290                 295                 300                 305

Ser Leu Asp Val Lys Gly Tyr Ser Leu Ala Glu Trp Ser Pro Glu Glu Phe Gly
            310                 315                 320

Lys Gly Gly Ser Ile Val Tyr Gln Lys Phe Thr Arg Thr Pro Phe Cys Gly Ser
325                 330                 335                 340

Pro Lys Ser Val Leu Lys Gly Cys Trp Lys Glu Asp Glu Glu Lys Glu Glu Lys
        345                 350                 355                 360

Glu
<210> 3
   <211> 1777
   <212> DNA
   <213> Populus deltoides
   <220>
   <400> SEQ ID NO: 3 
tcggacacaa aaaatattgc aagtttaggg tcataaaaat ctctatcatc acaaacaaat atagggtttg 70
ttgatttggt ggctgttttc gccgtctttg ctttcaaaat tcaatcttga cctgtttctt ttatgctttt 140
tgaataggtt tttttttga acttgagaaa gagggggttt tcaaaggaga ctagcttttc aagatttgat 210
atattttatt ggactgaatg atctaatgga gtctaaaggt ggcaagaaga agtctagtag tagtagtagt 280
agtaaatcct tatgttacga agctcccctc ggttacagca ttgaagacct cagacctgct ggtggaatca 350
agaagttcag atctgctgca tactccaact gcgttcgaaa accatcctga gattttccaa gtgttgacat 420
aaccccattt tagctatttc gcacgctcaa ttgtctttag tctgtttttc tgttctgctt tcctcgttct 490
cttgtactct ttgctgcact tttcatttgt tgactgtgag gtcagatggc gctgccagtc tctgcaatcg 560
gatttgaagg ttacgaaaaa aggcttgaaa tatctttcct agagcctggc ttcttttctg accctgaagg 630
gaagggcctg aggtctttgt ccaaggctca attggacgag attctcagac cagctgaatg tactattgtt 700
gattcgctat caaatgacca ggttgattct tatgtcctgt cggaatccag tctctttgtg tacccttaca 770
aagttattat caaaacatgt gggactacca aactgcttct ctctataccg gtgattctta agctcgctga 840
tgccctttca ctcactgtat gttctgtgag gtatactcgt gggagctttc tatgccctgg ggctcagcca 910
tttccacacc gcaacttctg tgaggaggta gctgtccttg atggccattt cagtaaactt ggtttgaaca 980
gtgtggcata tgtgatgggt ggtcttgaca aaactcagaa atggcatgtt tactctgcct ctgccgatat 1050
agagagccat tctggccctg tttacactct ggaaatgtgc atgactggtt tgggcaggaa acaagcatct 1120
gttttctaca aaacacattc cagttcagct gctgcgatga ctgaggattc cggcataagg aaaatccttc 1190
cacagtctga gatctgtgat tttgattttg acccttgtgg ttactctatg aatgccattg aagggagtgc 1260
aatttccaca atccacgtca ctccagaaga tggtttcagc tatgcaagtt ttgaggctgt gggctatgat 1330
cttcaagatt tgaatttgag tcagctgctt gaaagggttt tggcttgctt tgaaccgacc gagttctccg 1400
ttgctttgca ttctaatatc aagggtgccg aacttcgagc aaagtttccc ctggatgtgg aaggttactc 1470
tggtggagga gggaactatg aaatgcttgg gaaaggagga tcgatcatct accacagctt tgcaaggact 1540
ggaggcagtg catctcccag gtctatcctg aaatgttgtt ggagtgagga tgagaaggac gaggaagctg 1610
aagagaagta gttcttttca gctatttgtt ttttcctttt atttttttcct tgaataaatt tcatggggtt 1680
atgattctga gttcttagag gcatttgtcc atgccttgtg tctttcatta tcaatttagt tttgactttg 1750
gattaataaa ggggtttgtg ttatcag 1777
<210> 4
   <211> 361
   <212> PRT
   <213> Populus deltoides
   <400> SEQ ID NO: 4 
Met Ala Leu Pro Val Ser Ala Ile Gly Phe Glu Gly Tyr Glu Lys Arg Leu Glu
1               5                   10                  15
Ile Ser Phe Leu Glu Pro Gly Phe Phe Ser Asp Pro Glu Gly Lys Gly Leu Arg
    20                  25                  30                  35
 
Ser Leu Ser Lys Ala Gln Leu Asp Glu Ile Leu Arg Pro Ala Glu Cys Thr Ile
            40                  45                  50

Val Asp Ser Leu Ser Asn Asp Gln Val Asp Ser Tyr Val Leu Ser Glu Ser Ser
55                  60                  65                  70

Leu Phe Val Tyr Pro Tyr Lys Val Ile Ile Lys Thr Cys Gly Thr Thr Lys Leu
        75                  80                  85                  90

Leu Leu Ser Ile Pro Val Ile Leu Lys Leu Ala Asp Ala Leu Ser Leu Thr Val
                95                  100                 105

Cys Ser Val Arg Tyr Thr Arg Gly Ser Phe Leu Cys Pro Gly Ala Gln Pro Phe
    110                 115                 120                 125

Pro His Arg Asn Phe Cys Glu Glu Val Ala Val Leu Asp Gly His Phe Ser Lys
            130                 135                 140

Leu Gly Leu Asn Ser Val Ala Tyr Val Met Gly Gly Leu Asp Lys Thr Gln Lys
145                 150                 155                 160

Trp His Val Tyr Ser Ala Ser Ala Asp Ile Glu Ser His Ser Gly Pro Val Tyr
        165                 170                 175                 180

Thr Leu Glu Met Cys Met Thr Gly Leu Gly Arg Lys Gln Ala Ser Val Phe Tyr
                185                 190                 195

Lys Thr His Ser Ser Ser Ala Ala Ala Met Thr Glu Asp Ser Gly Ile Arg Lys
    200                 205                 210                 215

Ile Leu Pro Gln Ser Glu Ile Cys Asp Phe Asp Phe Asp Pro Cys Gly Tyr Ser
            220                 225                 230

Met Asn Ala Ile Glu Gly Ser Ala Ile Ser Thr Ile His Val Thr Pro Glu Asp
235                 240                 245                 250

Gly Phe Ser Tyr Ala Ser Phe Glu Ala Val Gly Tyr Asp Leu Gln Asp Leu Asn
        255                 260                 265                 270

Leu Ser Gln Leu Leu Glu Arg Val Leu Ala Cys Phe Glu Pro Thr Glu Phe Ser
                275                 280                 285

Val Ala Leu His Ser Asn Ile Lys Gly Ala Glu Leu Arg Ala Lys Phe Pro Leu
    290                 295                 300                 305

Asp Val Glu Gly Tyr Ser Gly Gly Gly Asn Tyr Glu Met Leu Gly Lys Gly
            310                 315                 320

Gly Ser Ile Ile Tyr His Ser Phe Ala Arg Thr Gly Gly Ser Ala Ser Pro Arg
325                 330                 335                 340

Ser Ile Leu Lys Cys Cys Trp Ser Glu Asp Glu Lys Asp Glu Glu Ala Glu Glu
        345                 350                 355                 360

Lys
<210> 5
   <211> 1763
   <212> DNA
   <213> Populus deltoides
   <220>
   <400> SEQ ID NO: 5 
acaaacaaga acccttcacg ttctttaaca atcttgcaga tttaagctct taaaaatctc tctcttcaaa 70

aacaaatcta gagtttgttg attttgtcat tggttttgcc cttttttttt ctataaaaaa ccaatcttga 140

tttgtttctt gaacagagtt tagtttggag tttgagaaag agggagtttt tgaaggagat taatttttca 210

agatttcata taatttattg acaagactga atgatccatg gagtctaaag gtggcaagaa gtctagtagt 280

agtagtagta gtagtaaatc cttattgtac gaagcacctc tcggctacag cattgaagac atcagacctg 350

ccggtggaat caagaagttc cgatctgctg cttactccaa ctgcgttcgg aagccatcct gagattttcc 420
 
aagtgttgag ataacccctt tttagctttt tcatactgtc tgttgtcctt agtctttttt tcttttctgc 490
tttcctcgtt ctctcgcact cttttctgca attgtcatct gtttactgtg aggtgagatg gcactgccgg 560
tctctgcaat cggatttgaa ggttacgaaa aaaggcttga aatttgcttt ttagagcctg gcttcttttc 630
tgaccctgaa ggaaagggcc tcaggtcttt gtccaaggct caattggacg agattctcaa accagctgaa 700
tgcactatag ttgattcgct ttcaaatgac gaggttgatt cgtatgttct gtcggaatcc agtctctttg 770
tataccctta caaagttatc atcaaaactt gtgggactac caaactgctt ctttcgatcc cagtgatcct 840
tgagctcgct gatgcccttt cactcactgt atgttctgtg aggtatactc gtgggagctt catatgtccc 910
ggggcgcagc catttccgca tcgtaacttc tgtgaggagg taactgtcct cgatggccat ttcagtaaat 980
ttggtttaga gagtgtggca tatgtgatgg gaagtcccaa ctcaactcag aaatggcatg tttactctgc 1050
tgctgctggt gtgaagagcc attctggccc tgtttacact ctggaaatgt gcatgactgg tttggacagg 1120
aagcgagcat ctgttttcta caaaacacac gccagttcag ctactgttat gactgaggat tctggtataa 1190
ggaagatcct tccgcaatct gagatctgcg attttgattt tgacccttgt ggttactcta tgaatgccat 1260
tgaagggagt gcaatttcca caatccatgt caccccagaa gatggtttca gctatgcaag ttttgaggct 1330
gtgggttatg attttcaaga attaaatttg aagcagctcc tttatagggt tttggtttgc tttgaaccga 1400
ccgagttctc cattgcgttg cattctaatg ttgagtgtga cgaacttgga gcgatgtttt ccctggatgt 1470
gaaaggttac tcttgtggag gggggaacta tgaaatgctc gggaagggtg gatcgattgt ctaccacagc 1540
tttgcagcga ctggaggctg ctcatctccc aggtcaatcc tgaaatgttg ttggagtgag gacgaggaag 1610
ctgaagagaa gtagttcttt tcagcaatgt gttttttctt tttcttttct tttttgtgtt tagttgttga 1680
tgtcatgggg ttatgattct gagttttaga ggcatttgtc catgccttga gtctttcatt acaatttatc 1750
tcatgtcttg ttc 1763
<210> 6
   <211> 358
   <212>.PRT
   <213> Populus deltoides
   <400> SEQ ID NO: 6 
Met Ala Leu Pro Val Ser Ala Ile Gly Phe Glu Gly Tyr Glu Lys Arg Leu Glu
1               5                   10                  15

Ile Cys Phe Leu Glu Pro Gly Phe Phe Ser Asp Pro Glu Gly Lys Gly Leu Arg
    20                  25                  30                  35

Ser Leu Ser Lys Ala Gln Leu Asp Glu Ile Leu Lys Pro Ala Glu Cys Thr Ile
            40                  45                  50

Val Asp Ser Leu Ser Asn Asp Glu Val Asp Ser Tyr Val Leu Ser Glu Ser Ser
55                  60                  65                  70

Leu Phe Val Tyr Pro Tyr Lys Val Ile Ile Lys Thr Cys Gly Thr Thr Lys Leu
        75                  80                  85                  90

Leu Leu Ser Ile Pro Val Ile Leu Glu Leu Ala Asp Ala Leu Ser Leu Thr Val
                95                  100                 105

Cys Ser Val Arg Tyr Thr Arg Gly Ser Phe Ile Cys Pro Gly Ala Gln Pro Phe
    110                 115                 120                 125

Pro His Arg Asn Phe Cys Glu Glu Val Thr Val Leu Asp Gly His Phe Ser Lys
            130                 135                 140

Phe Gly Leu Glu Ser Val Ala Tyr Val Met Gly Ser Pro Asn Ser Thr Gln Lys
145                 150                 155                 160
 
Trp His Val Tyr Ser Ala Ala Ala Gly Val Lys Ser His Ser Gly Pro Val Tyr
        165                 170                 175                 180

Thr Leu Glu Met Cys Met Thr Gly Leu Asp Arg Lys Arg Ala Ser Val Phe Tyr
                185                 190                 195

Lys Thr His Ala Ser Ser Ala Thr Val Met Thr Glu Asp Ser Gly Ile Arg Lys
    200                 205                 210                 215

Ile Leu Pro Gln Ser Glu Ile Cys Asp Phe Asp Phe Asp Pro Cys Gly Tyr Ser
            220                 225                 230

Met Asn Ala Ile Glu Gly Ser Ala Ile Ser Thr Ile His Val Thr Pro Glu Asp
235                 240                 245                 250

Gly Phe Ser Tyr Ala Ser Phe Glu Ala Val Gly Tyr Asp Phe Gln Glu Leu Asn
        255                 260                 265                 270

Leu Lys Gln Leu Leu Tyr Arg Val Leu Val Cys Phe Glu Pro Thr Glu Phe Ser
                275                 280                 285

Ile Ala Leu His Ser Asn Val Glu Cys Asp Glu Leu Gly Ala Met Phe Ser Leu
    290                 295                 300                 305

Asp Val Lys Gly Tyr Ser Cys Gly Gly Gly Asn Tyr Glu Met Leu Gly Lys Gly
            310                 315                 320

Gly Ser Ile Val Tyr His Ser Phe Ala Ala Thr Gly Gly Cys Ser Ser Pro Arg
325                 330                 335                 340

Ser Ile Leu Lys Cys Cys Trp Ser Glu Asp Glu Glu Ala Glu Glu Lys
        345                 350                 355
<210> 7
   <211> 2523
   <212> DNA
   <213> Populus deltoides
   <220>
   <400> SEQ ID NO: 7 
ttaaccctat atttctctca gaactcacat aaaaaatatt gcgagtttag ggtcataaaa atctctatca 70

tcacaaacaa atatagggtt tgttgatttg gtggctgttt tcgccgtctt tgctttcaaa attcaatctt 140

gacctgtttc ttttatgctt tttgaatagg taatatatac attgtttgtt ttgcttgctt tggttttgta 210

atctggtgat cttgtttaat tctttttggc tttggtttgt aggttttttt ttttgaacyt gagaaagagg 280

ggggttttca arggagacta gcttttcaag awttgatata ttttattggt aagtttttnt tttccgtttt 350

ttggtaatag ttgcatggat tcttggtgta gatgttgatt tggacttgtt tttgtattgt tgtcctttat 420

gttttatgtg tatgttaatt gttctgatcc ttgatataat gtaaatctga ggcttttatt tttatggatt 490

cgttaaggtg tatggacttg gtttatggtt ttggtcttga aaaaaaaatt aggcttagca agttttcttc 560

tgagaataac ttattttatt ttattattta aaattaaatg gtgtttggga atttgaattc tttctctttg 630

gtattttttc tatccttgtg aatctgttta gtcttttaaa ttcaatattt tattttattt tacttatgat 700

ttggtgattg attagttatt tttgtagctt gtattgtctg ttgattttta atctccaata tgtcttactt 770

ctcagttttt ttcaatttgc tattggaatt ttgctattat ttttaagatt gtagactagt ttgctttgag 840

ttttgtgcta attgcaatcc ttcttgtgtg attcttacag gactgaatga tctaatggag tctaaaggtg 910

gcaagaagaa gtctagtagt agtagtagta gtaaatcctt atgttacgaa gctcccctcg gttacagcat 980

tgaagacctc agacctgctg gtggaatcaa gaagttcaga tctgctgcat actccaacgt gagtttgaag 1050

ttaatcgatc tgtttttcct ttggcatttc attgtgcgac tggtttccta atcttgtgtc atgtttattt 1120

gtgcagtgcg ttcgaaaacc atcctgagat tttccaagtg ttgacataac cccattttag ctatttcgca 1190
 
cgctcagttg tctttaktct gtttttctgt tctgctttcc tcgttctctt gtactctttg ctgcactttt 1260

catttgttga ctgtgaggtc agatggcgct gccagtctct gcaatcggat ttgaaggtta cgaaaaaagg 1330

cttgaaatat ctttcctaga gcctggcttc ttttctgacc ctgaagggaa gggcctgagg tctttgtcca 1400

aggctcaatt ggacgagatt ctcagaccag ctgaatgtac tattgttgat tcgctatcaa atgaccaggt 1470

tgattcttat gtcctgtcgg aatccagtct ctttgtgtac ccttacaaag ttattatcaa aacatgtggg 1540

actaccaaac tgcttctctc gataccggtg attcttaagc tcgctgatgc cctttcactc actgtatgtt 1610

ctgtgaggta tactcgtggg agc[?]ttctat gccctggggc tcagccattt ccacaccgca acttctgtga 1680

ggaggtagct gtccttgacg atcacttcag taaacttggt ttaaacagtg tggcatatgt gatgggtggt 1750

cttgacaaaa ctcagaaatg gcatgtttac tctgcctctg ccgatataga gagccattct ggccctgttt 1820

acactctgga aatgtgcatg actggtttgg gcaggaaaca agcatctgtt ttctacaaaa cacattccag 1890

ttcagctgct gcgatgactg aggattccgg tataaggaaa atccttccac agtctgagat ctgtgatttt 1960

gattttgacc catgtggtta ctctatgaat gccattgaag ggagtgcaat ttccacaatc cacgtcactc 2030

cagaagatgg tttcagctat gcaagttttg aggctgtggg ctatgatctt caagatttga atttgagtcg 2100

gctgcttgaa agggtcttgg cttgctttga accgaccatg ttctccgttg ccttgcattc taatatcaag 2170

ggtgccgaac ttagagcaaa gtttcccctg gacgtggaag gttactctgg cggaggaggg aactatgaaa 2240

tgcttgggaa aggtggatcg atcatctacc acagctttgc aaggactgga ggcagtgcat ctcccaggtc 2310

tatcctgaaa tgttgttgga gtgaggatga gaaggacgag gaagctgaag agaagtagtt cttttcagct 2380

atttgttttt tcctttatt ttttccttga ataaatgtca tggggttatg attctgagtt cttgaggcat 2450

ttgtccatgc ctcgtgtctt tcattatcga tttagttttg acttcggatt aataaagggg tttgtgttat 2520

cag 2523
<210> 8
   <211> 361
   <212> PRT
   <213> Populus deltoides
   <400> SEQ ID NO: 8 
Met Ala Leu Pro Val Ser Ala Ile Gly Phe Glu Gly Tyr Glu Lys Arg Leu Glu
1               5                   10                  15

Ile Ser Phe Leu Glu Pro Gly Phe Phe Ser Asp Pro Glu Gly Lys Gly Leu Arg
    20                  25                  30                  35

Ser Leu Ser Lys Ala Gln Leu Asp Glu Ile Leu Arg Pro Ala Glu Cys Thr Ile
            40                  45                  50

Val Asp Ser Leu Ser Asn Asp Gln Val Asp Ser Tyr Val Leu Ser Glu Ser Ser
55                  60                  65                  70

Leu Phe Val Tyr Pro Tyr Lys Val Ile Ile Lys Thr Cys Gly Thr Thr Lys Leu
        75                  80                  85                  90

Leu Leu Ser Ile Pro Val Ile Leu Lys Leu Ala Asp Ala Leu Ser Leu Thr Val
                95                  100                 105

Cys Ser Val Arg Tyr Thr Arg Gly Ser Phe Leu Cys Pro Gly Ala Gln Pro Phe
    110                 115                 120                 125

Pro His Arg Asn Phe Cys Glu Glu Val Ala Val Leu Asp Asp His Phe Ser Lys
            130                 135                 140

Leu Gly Leu Asn Ser Val Ala Tyr Val Met Gly Gly Leu Asp Lys Thr Gln Lys
145                 150                 155                 160

Trp His Val Tyr Ser Ala Ser Ala Asp Ile Glu Ser His Ser Gly Pro Val Tyr
 
        165                 170                 175                 180

Thr Leu Glu Met Cys Met Thr Gly Leu Gly Arg Lys Gln Ala Ser Val Phe Tyr
                185                 190                 195

Lys Thr His Ser Ser Ser Ala Ala Ala Met Thr Glu Asp Ser Gly Ile Arg Lys
    200                 205                 210                 215

Ile Leu Pro Gln Ser Glu Ile Cys Asp Phe Asp Phe Asp Pro Cys Gly Tyr Ser
            220                 225                 230

Met Asn Ala Ile Glu Gly Ser Ala Ile Ser Thr Ile His Val Thr Pro Glu Asp
235                 240                 245                 250

Gly Phe Ser Tyr Ala Ser Phe Glu Ala Val Gly Tyr Asp Leu Gln Asp Leu Asn
        255                 260                 265                 270

Leu Ser Arg Leu Leu Glu Arg Val Leu Ala Cys Phe Glu Pro Thr Met Phe Ser
                275                 280                 285

Val Ala Leu His Ser Asn Ile Lys Gly Ala Glu Leu Arg Ala Lys Phe Pro Leu
    290                 295                 300                 305

Asp Val Glu Gly Tyr Ser Gly Gly Gly Gly Asn Tyr Glu Met Leu Gly Lys Gly
            310                 315                 320

Gly Ser Ile Ile Tyr His Ser Phe Ala Arg Thr Gly Gly Ser Ala Ser Pro Arg
325                 330                 335                 340

Ser Ile Leu Lys Cys Cys Trp Ser Glu Asp Glu Lys Asp Glu Glu Ala Glu Glu
        345                 350                 355                 360

Lys
<210> 9
   <211> 1878
   <212> DNA
   <213> Arabidopsis thaliana
   <220>
   <400> SEQ ID NO: 9 
gaacgcatct cattgcttca tcattaccaa atcatcaact taatcgtttc tctcaaattt agggttttct 70

cttttctcga aagtcttgcg gttttctgaa tcatctctat ctggtttgag ggtttcgttt gatatctgga 140

gaaaggggtt tctggaaaca aggagttcat aattcgcgat cttgatctat cgatcttcat ttatatataa 210

aagcgtgaat gagattatga tggagtcgaa aggtggtaaa aagaagtcca gcagtagtag ttccttattt 280

tacgaagctc ccctcggtta cagcattgaa gacgttcgtc caaacggtgg aatcaagaaa ttcaaatctt 350

ctgtctactc aaactgctcc aagaggccat cctgagtacc agcgtgcacc gatcttcata atatagttat 420

agctttcttt actttccagt ttataatttt cttctttcaa agctcctttt ctgctggttc ccggatccaa 490

tcgttctctc ctcctactac aagtcctgtc gctcacacaa caaggcgaga tggccttatc tgcaatcggt 560

ttcgaaggtt acgagaaacg gctcgaggtg actttctttg agccaagcat ctttcaagac tccaagggac 630

tgggactccg tgctctgacc aagtcccagc ttgatgaaat tcttacacct gctgcatgca cgatcgtttc 700

atctctctcc aacgatcaat tggactctta cgtactctct gagtccagct tctttgtcta cccctacaaa 770

gtcatcatca agacttgcgg taccactaag ctcctcctct ctatcccacc acttctaaag ctggctggtg 840

agctctctct gagtgtcaag tctgtgaagt acactcgcgg ctccttcctc tgccccggag gccagccttt 910

tcctcaccgc agcttctctg aagaagtctc tgttcttgat gggcacttta ctcagctggg cttgaacagc 980

gtagcctact tgatgggcaa tgatgatgag actaagaaat ggcatgtcta tgctgcctct gcccaggact 1050

ccagcaactg caacaacaat gtctacactc tcgagatgtg catgactggt ctggacagag agaaagctgc 1120

tgtcttctac aaggatgaag ctgacaagac tgggtcaatg actgataact ctggaatcag aaagatcctt 1190
 
cccaagtctg agatctgcga ctttgaattc gagccctgcg gctactctat gaactcaatt gaaggggatg 1260
caatctccac gatccatgtg acccctgaag atgggtttag ctacgctagc ttcgaagctg tgggttacga 1330
cttcaacacc cttgacctta gccagctggt gacaagggtt ctctcttgct tcgagcccaa gcaattctct 1400
gtagctgtgc actcgagcgt tggagcgaac tcatacaagc cagagattac tgtagacttg gaagactatg 1470
ggtgcagaga gaggacattt gagtctctag gagaagagag tggaacagtg atgtatcaga cgtttgagaa 1540
gcttggtaag tactgtggat cgcctagatc taccttgaag tgtgaatgga gcagcaacaa tagctgcagc 1610
agcgaggacg agaaggacga gggaatctag cagaattttt cttcctaata actattttcg agctttctgt 1680
ttttgttctt tctttttaaa aaacttatta agttcttatg aataatgact tgtgaagttt gagttcgtct 1750
ccttcacaag caagttgtat tggtgttttc tactttatga atatgggttt tatataccta aagacttgtt 1820
atgttattat tcttaaatgt tgctgctatg atgattacta ttatcgattt ttactaaa 1878
<210> 10
   <211> 366
   <212> PRT
   <213> Arabidopsis thaliana
   <400> SEQ ID NO: 10 
Met Ala Leu Ser Ala Ile Gly Phe Glu Gly Tyr Glu Lys Arg Leu Glu Val Thr
1               5                   10                  15

Phe Phe Glu Pro Ser Ile Phe Gln Asp Ser Lys Gly Leu Gly Leu Arg Ala Leu
    20                  25                  30                  35

Thr Lys Ser Gln Leu Asp Glu Ile Leu Thr Pro Ala Ala Cys Thr Ile Val Ser
            40                  45                  50

Ser Leu Ser Asn Asp Gln Leu Asp Ser Tyr Val Leu Ser Glu Ser Ser Phe Phe
55                  60                  65                  70

Val Tyr Pro Tyr Lys Val Ile Ile Lys Thr Cys Gly Thr Thr Lys Leu Leu Leu
        75                  80                  85                  90

Ser Ile Pro Pro Leu Leu Lys Leu Ala Gly Glu Leu Ser Leu Ser Val Lys Ser
                95                  100                 105

Val Lys Tyr Thr Arg Gly Ser Phe Leu Cys Pro Gly Gly Gln Pro Phe Pro His
    110                 115                 120                 125

Arg Ser Phe Ser Glu Glu Val Ser Val Leu Asp Gly His Phe Thr Gln Leu Gly
            130                 135                 140

Leu Asn Ser Val Ala Tyr Leu Met Gly Asn Asp Asp Glu Thr Lys Lys Trp His
145                 150                 155                 160

Val Tyr Ala Ala Ser Ala Gln Asp Ser Ser Asn Cys Asn Asn Asn Val Tyr Thr
        165                 170                 175                 180

Leu Glu Met Cys Met Thr Gly Leu Asp Arg Glu Lys Ala Ala Val Phe Tyr Lys
                185                 190                 195

Asp Glu Ala Asp Lys Thr Gly Ser Met Thr Asp Asn Ser Gly Ile Arg Lys Ile
    200                 205                 210                 215

Leu Pro Lys Ser Glu Ile Cys Asp Phe Glu Phe Glu Pro Cys Gly Tyr Ser Met
            220                 225                 230

Asn Ser Ile Glu Gly Asp Ala Ile Ser Thr Ile His Val Thr Pro Glu Asp Gly
235                 240                 245                 250

Phe Ser Tyr Ala Ser Phe Glu Ala Val Gly Tyr Asp Phe Asn Thr Leu Asp Leu
        255                 260                 265                 270

Ser Gln Leu Val Thr Arg Val Leu Ser Cys Phe Glu Pro Lys Gln Phe Ser Val
                275                 280                 285
 
Ala Val His Ser Ser Val Gly Ala Asn Ser Tyr Lys Pro Glu Ile Thr Val Asp
    290                 295                 300                 305

Leu Glu Asp Tyr Gly Cys Arg Glu Arg Thr Phe Glu Ser Leu Gly Glu Glu Ser
            310                 315                 320

Gly Thr Val Met Tyr Gln Thr Phe Glu Lys Leu Gly Lys Tyr Cys Gly Ser Pro
325                 330                 335                 340

Arg Ser Thr Leu Lys Cys Glu Trp Ser Ser Asn Asn Ser Cys Ser Ser Glu Asp
        345                 350                 355                 360

Glu Lys Asp Glu Gly Ile
                365
<210> 11
   <211> 795
   <212> DNA
   <213> Xanthomonas axonopodis pv. citri
   <220>
   <400> SEQ ID NO: 11 
atggtcaagc cgttgcctcg tctgaggcta caggggttca acaacctcac caaggcgttg agcttcaaca 70

tctacgacgt ctgttatgcg cgtaccgaag aggagcgtca gcgctacatc gagtatatcg atgagcaata 140

cgacgccgat cgtctgacgc agatcctgac cgatgtggcc gagatcattg gcgccaacat cctcaatatc 210

gcacgtcagg actacgatcc gcagggtgca tcggtgacga tcctgatctc cgaagagccg gtgatcgaca 280

agaagcaggc cggcaaggaa ttgatctccg atgccgtggt tgctcatatg gataagagtc atatcactgt 350

ccatacatat ccggaaacgc atccgcagga aggcatcgcc acgttccgcg ccgacatcga cgtcgcgacc 420

tgcggcgtga tttcgccgtt gaaagcgctc aactacctga tcgagagtct ggaatcggac attgtgatca 490

tggattaccg cgtgcgtggc ttcacgcgcg atgtgaaggg caagaagcat tacatcgatc acaagatcaa 560

ctcgattcag caatttctcg ccaagaacgt gaagtcgcgc tacgagatgt tcgacgtcaa cgtctaccag 630

gaaaatatct tccacacgaa gatgcacctg aaggacttcg acctggatca gtacctgttc gaagagcgcg 700

ccaagaacct ctcgttcaag gagcgcatga agatcgaaac gctgctcaag cgcgaaatcg aagaactgtt 770

tcacggacgc aacctgagcg aataa 795
<210> 12
   <211> 264
   <212> PRT
   <213> Xanthomonas axonopodis pv. citri
   <400> SEQ ID NO: 12 
Met Val Lys Pro Leu Pro Arg Leu Arg Leu Gln Gly Phe Asn Asn Leu Thr Lys
1               5                   10                  15

Ala Leu Ser Phe Asn Ile Tyr Asp Val Cys Tyr Ala Arg Thr Glu Glu Glu Arg
    20                  25                  30                  35

Gln Arg Tyr Ile Glu Tyr Ile Asp Glu Gln Tyr Asp Ala Asp Arg Leu Thr Gln
            40                  45                  50

Ile Leu Thr Asp Val Ala Glu Ile Ile Gly Ala Asn Ile Leu Asn Ile Ala Arg
55                  60                  65                  70

Gln Asp Tyr Asp Pro Gln Gly Ala Ser Val Thr Ile Leu Ile Ser Glu Glu Pro
        75                  80                  85                  90

Val Ile Asp Lys Lys Gln Ala Gly Lys Glu Leu Ile Ser Asp Ala Val Val Ala
                95                  100                 105

His Met Asp Lys Ser His Ile Thr Val His Thr Tyr Pro Glu Thr His Pro Gln
 
110                 115                 120                 125

Glu Gly Ile Ala Thr Phe Arg Ala Asp Ile Asp Val Ala Thr Cys Gly Val Ile
        130                 135                 140

Ser Pro Leu Lys Ala Leu Asn Tyr Leu Ile Glu Ser Leu Glu Ser Asp Ile Val
145                 150                 155                 160

Ile Met Asp Tyr Arg Val Arg Gly Phe Thr Arg Asp Val Lys Gly Lys Lys His
        165                 170                 175                 180

Tyr Ile Asp His Lys Ile Asn Ser Ile Gln Gln Phe Leu Ala Lys Asn Val Lys
                185                 190                 195

Ser Arg Tyr Glu Met Phe Asp Val Asn Val Tyr Gln Glu Asn Ile Phe His Thr
    200                 205                 210                 215

Lys Met His Leu Lys Asp Phe Asp Leu Asp Gln Tyr Leu Phe Glu Glu Arg Ala
        220                 225                 230

Lys Asn Leu Ser Phe Lys Glu Arg Met Lys Ile Glu Thr Leu Leu Lys Arg Glu
235                 240                 245                 250

Ile Glu Glu Leu Phe His Gly Arg Asn Leu Ser Glu
        255                 260
<210> 13
   <211> 1820
   <212> DNA
   <213> Datura stramonium
   <220>
   <400> SEQ ID NO: 13 
cttgtatact cttccgaatc gccttctcca aatttctagg gtttctctct tcttcggaat tttctggttt 70
ggaaagatcg ttgtttattg gagataaatc ggatttggaa caaatttcaa tttttaaata ttgtgaatga 140
tctaatggag tcaaaaggtg ggaaaaagaa gtctagtagt agttccttat tttacgaagc tcccctcggc 210
tacagcattg aagacgttcg accaaacggt ggagtgaaga agttcagatc tgctgcttac tccaactgcg 280
cgcgcaaacc atcctgatat tccctaaact tctgtcctta aagcgtcaat agacgcaacc aaaaaaaaca 350
aaaaaaattt ctgctttcaa tttctttcgt tgtcaagccc tcactccttt tcttccttct tttactactt 420
cctgctttgc actcgttgct ctgaacattt tctgctttaa cttcttttgc tgctgtgaac tttttttcata 490
atggaaatgg acttgccagt ctctgccatc ggttttgaag gttttgaaaa gaggctcgaa atttcttttg 560
tcgagcctgg tctgttttct gatcctaatg gaaaaggact tcgatctctc tcaaaggcac agttggatga 630
aattctcgga cctgctgagt gcaccattgt cgataaccta tcaaatgact atgttgattc ctatgtcctc 700
tctgagtcga gcctcttcgt ttattcttac aagataatca ttaaaacatg tggcaccaca aagttgcttc 770
tcgcaattcc gcccattcta aggttggctg agaccttgtc tctcaaagta caagatgtga ggtatacccg 840
tgggagcttc attttccctg gtgctcaatc gtttcctcac cgtcactttt ctgaagaagt tgctgtcctc 910
gatggctatt ttgggaagct tgctgccggt agcaaggctg tgattatggg caaccctgac aaaacacaga 980
aatggcatgt ttactctgcc tcagctgggc ctgttcagtc taatgaccct gtttacactc tagagatgtg 1050
tatgactggt ttggacaggg agaaggcatc tgtcttttac aagactgaag gaagctcggc tgctcacatg 1120
actgttagat ctggcataag gaagatcctc cccaactctg agatatgcga ttttgagttt gaaccctgtg 1190
gttattccat gaattctatt gaaggagctg ctgtttcaac cattcacatt acaccagaag acggctttag 1260
ctatgctagc tttgaatcag ttggatatga tctgaaaacc atggagttgg gtcccctggt tgagagggtg 1330
ctggcatgtt ttgagcctgc agagttctct attgctttgc atgctgatgt tgctaccaag ttactggagc 1400
gtgtttgctg tgttgatgtt aagggctact ctcttgctga gtggagtcca gaagaatttg gcaagggtgg 1470
 
ttccattgtc taccagaagt tcactaagac tccttactgt gcatctccca agtccgttct gaagggctgc 1540
tggaaagagg aagaggaaga gaaagaagaa aaggagtagt gtttgtcttg agggtcgttt tgttattgtt 1610
gttttagtgt ctgtcgtttg ctcatgtttt acctgtttgt cagaataaag gacttaagat tgtcccattt 1680
gtgtcagttt ggatttgtaa cgattgtgtg caaattctga actagtccgg cttcttggtg ttccaccaga 1750
agccctatgt gtctgcattt tgttccctgt tccgttgtgg cttctaataa aagtttgttt gttgtgtgta 1820
<210> 14
   <211> 362
   <212> PRT
   <213> Datura stramonium
   <400> SEQ ID NO: 14 
Met Glu Met Asp Leu Pro Val Ser Ala Ile Gly Phe Glu Gly Phe Glu Lys Arg
1               5                   10                  15

Leu Glu Ile Ser Phe Val Glu Pro Gly Leu Phe Ser Asp Pro Asn Gly Lys Gly
    20                  25                  30                  35

Leu Arg Ser Leu Ser Lys Ala Gln Leu Asp Glu Ile Leu Gly Pro Ala Glu Cys
            40                  45                  50

Thr Ile Val Asp Asn Leu Ser Asn Asp Tyr Val Asp Ser Tyr Val Leu Ser Glu
55                  60                  65                  70

Ser Ser Leu Phe Val Tyr Ser Tyr Lys Ile Ile Ile Lys Thr Cys Gly Thr Thr
        75                  80                  85                      90

Lys Leu Leu Leu Ala Ile Pro Pro Ile Leu Arg Leu Ala Glu Thr Leu Ser Leu
                95                  100                 105

Lys Val Gln Asp Val Arg Tyr Thr Arg Gly Ser Phe Ile Phe Pro Gly Ala Gln
    110                 115                 120                 125

Ser Phe Pro His Arg His Phe Ser Glu Glu Val Ala Val Leu Asp Gly Tyr Phe
            130                 135                 140

Gly Lys Leu Ala Ala Gly Ser Lys Ala Val Ile Met Gly Asn Pro Asp Lys Thr
145                 150                 155                 160

Gln Lys Trp His Val Tyr Ser Ala Ser Ala Gly Pro Val Gln Ser Asn Asp Pro
    165                 170                 175                     180

Val Tyr Thr Leu Glu Met Cys Met Thr Gly Leu Asp Arg Glu Lys Ala Ser Val
                185                 190                 195

Phe Tyr Lys Thr Glu Gly Ser Ser Ala Ala His Met Thr Val Arg Ser Gly Ile
    200                 205                 210                 215

Arg Lys Ile Leu Pro Asn Ser Glu Ile Cys Asp Phe Glu Phe Glu Pro Cys Gly
            220                 225                 230

Tyr Ser Met Asn Ser Ile Glu Gly Ala Ala Val Ser Thr Ile His Ile Thr Pro
235                 240                 245                 250

Glu Asp Gly Phe Ser Tyr Ala Ser Phe Glu Ser Val Gly Tyr Asp Leu Lys Thr
    255                 260                 265                     270

Met Glu Leu Gly Pro Leu Val Glu Arg Val Leu Ala Cys Phe Glu Pro Ala Glu
                275                 280                 285

Phe Ser Ile Ala Leu His Ala Asp Val Ala Thr Lys Leu Leu Glu Arg Val Cys
    290                 295                 300                 305

Cys Val Asp Val Lys Gly Tyr Ser Leu Ala Glu Trp Ser Pro Glu Glu Phe Gly
            310                 315                 320

Lys Gly Gly Ser Ile Val Tyr Gln Lys Phe Thr Lys Thr Pro Tyr Cys Ala Ser
325                 330                 335                 340
 
Pro Lys Ser Val Leu Lys Gly Cys Trp Lys Glu Glu Glu Glu Glu Lys Glu Glu
        345                 350                 355                 360
Lys Glu .
<210> 15
   <211> 1971
   <212> DNA
   <213> Oryza sativa
   <220>
   <400> SEQ ID NO: 15 
acgcgtccgt ttcgttttgg tattcccata cactttgttt ctcttccccg tctcgtggtc gagagaaacc 70
gagaggagag agagggagag gagcgctgcc gattttccgg agggggagat ctttttcttc tccctgaagg 140
ttcgagatcg atctgcgacg aggaggagcg gggaatcgta actttatttc cgaggaaatt tcaacaaatt 210
gatgcactaa tggagtccaa gggtggcaaa aagaagtcta gcagtagtcg ttcctccctg atgtacgaag 280
ctcccctcgg ctacagcatt gaggacctcc gacctgccgg cggcatcaag aagttccgct ctgctgctta 350
ctccaactgc gcgaggaagc cctcttgata gcccccttgg ctaccttgac ctagtagttt agttactcct 420
ctgaattctc gttttgggct ttcttacatc tctctggcta gctgcttccc agtgaccggg aaggtcatca 490
gtcctgcttc ctttcttcct catctctggc atcgtccttc gaaacaatgg cagtcctgtc agttgctgac 560
tctccaccgg tctcggcgat cgggtttgag ggatatgaga aacgcctcga gatcactttc tccgaggcgc 630
ctgtctttgc tgaccccaat ggcagggggt tgcgtgcact gtcacgtgct cagattgact ctgttcttga 700
tctggcgagg tgcaccattg tgtctgagct ctccaatgag gtcttcgact cgtatgtcct ttctgaatcg 770
agcctctttg tgtacccata caagattgtg atcaagacct gtgggactac caagctcctg ctcgccattc 840
cgaggattct tgagcttgct gaagagctat ctctgccact tgaagctgtg aagtactctc gtgggacatt 910
cattttccct gaagcacagc cctctccaca caagaacttc tctgaggagg ttgcagtcct gaaccgctac 980
ttcggcggtc tcaaatctgg tggcaacgca tacgtgattg gagatcctgc aaagccaggg cagaagtggc 1050
atgtctacta tgccacccag cacccggagc aacctgtggt cactcttgag atgtgcatga ctgggctgga 1120
caagaagaaa gcttctgtct tcttcaagac ttctgctgat ggacacacaa catatgctaa ggaaatgacc 1190
aagctctcag gtatctctga catcatccca gagatggaag tctgcgactt cgatttcgag ccctgcggct 1260
actccatgaa tgccatccac ggccctgctt tctcaaccat tcatgtgact cctgaggatg gcttcagcta 1330
tgccagctac gaggtcatgg gcttcaaccc tgcttccctt gcctatggtg accttgtcaa gagggtgttg 1400
agatgctttg gcccgttgga gttctctgtt gcggttacca tctttggtgg ccgcaaccat gcagggacct 1470
gggccaaggg gctggatgtc ggggcctatt cttgcagcaa catggttgag caggagctgc cttctggggg 1540
tttgctcatt taccagagct tcactgctac tgctgaaatc gccaccgggt cgccacgatc tgttctgcat 1610
tgctttgctg atgaaaacac ggagaaagct ggtaaaatgg aggctctcta ctgggaagac gatgctgtcg 1680
aggagataga tggcacagag ggtaagaaga tgaggagctg ctgatgaggg aagcgcactg aagattaaag 1750
aatactcaaa actccagtag cgatcactct gttactttgt gaagcagcca gccagactat tcatatagta 1820
ctagtatatg ctatgaccaa tgttctttat tatcttgcat atattctgaa taaaagggct ctaggtggtc 1890
tgctttgtct gccacagtga gcaaaatgta ttcgaatatc aaactttaat gttgggaata taatacaagt 1960
cattttataa t 1971
<210> 16
   <211> 395
   <212> PRT
   <213> Oryza sativa <400> SEQ ID NO: 16 
Met Ala Val Leu Ser Val Ala Asp Ser Pro Pro Val Ser Ala Ile Gly Phe Glu
1               5                   10                  15

Gly Tyr Glu Lys Arg Leu Glu Ile Thr Phe Ser Glu Ala Pro Val Phe Ala Asp
    20                  25                  30                  35

Pro Asn Gly Arg Gly Leu Arg Ala Leu Ser Arg Ala Gln Ile Asp Ser Val Leu
            40                  45                  50

Asp Leu Ala Arg Cys Thr Ile Val Ser Glu Leu Ser Asn Glu Val Phe Asp Ser
55                  60                  65                  70

Tyr Val Leu Ser Glu Ser Ser Leu Phe Val Tyr Pro Tyr Lys Ile Val Ile Lys
        75                  80                  85                      90

Thr Cys Gly Thr Thr Lys Leu Leu Leu Ala Ile Pro Arg Ile Leu Glu Leu Ala
                95                  100                 105

Glu Glu Leu Ser Leu Pro Leu Glu Ala Val Lys Tyr Ser Arg Gly Thr Phe Ile
    110                 115                 120                 125

Phe Pro Glu Ala Gln Pro Ser Pro His Lys Asn Phe Ser Glu Glu Val Ala Val
            130                 135                 140

Leu Asn Arg Tyr Phe Gly Gly Leu Lys Ser Gly Gly Asn Ala Tyr Val Ile Gly
145                 150                 155                 160

Asp Pro Ala Lys Pro Gly Gln Lys Trp His Val Tyr Tyr Ala Thr Gln His Pro
        165                 170                 175                     180

Glu Gln Pro Val Val Thr Leu Glu Met Cys Met Thr Gly Leu Asp Lys Lys Lys
                185                 190                 195

Ala Ser Val Phe Phe Lys Thr Ser Ala Asp Gly His Thr Thr Tyr Ala Lys Glu
    200                 205                 210                 215

Met Thr Lys Leu Ser Gly Ile Ser Asp Ile Ile Pro Glu Met Glu Val Cys Asp
            220                 225                 230

Phe Asp Phe Glu Pro Cys Gly Tyr Ser Met Asn Ala Ile His Gly Pro Ala Phe
235                 240                 245                 250

Ser Thr Ile His Val Thr Pro Glu Asp Gly Phe Ser Tyr Ala Ser Tyr Glu Val
        255                 260                 265                     270

Met Gly Phe Asn Pro Ala Ser Leu Ala Tyr Gly Asp Leu Val Lys Arg Val Leu
                275                 280                 285

Arg Cys Phe Gly Pro Leu Glu Phe Ser Val Ala Val Thr Ile Phe Gly Gly Arg
    290                 295                 300                 305

Asn His Ala Gly Thr Trp Ala Lys Gly Leu Asp Val Gly Ala Tyr Ser Cys Ser
            310                 315                 320

Asn Met Val Glu Gln Glu Leu Pro Ser Gly Gly Leu Leu Ile Tyr Gln Ser Phe
325                 330                 335                 340

Thr Ala Thr Ala Glu Ile Ala Thr Gly Ser Pro Arg Ser Val Leu His Cys Phe
        345                 350                 355                     360

Ala Asp Glu Asn Thr Glu Lys Ala Gly Lys Met Glu Ala Leu Tyr Trp Glu Asp
                365                 370                 375

Asp Ala Val Glu Glu Ile Asp Gly Thr Glu Gly Lys Lys Met Arg Ser Cys
    380                 385                 390                 395
<210> 17
   <211> 2952
   <212> DNA
   <213> Oryza sativa
   <220>
   <400> SEQ ID NO: 17 
gcctgggcga ttctagaaag atgtctcttt tgctaatggt tattattagc tgaatcgctg atatggtgaa 70
tccaggaaag tttcatgctt tttgtcctat gtcctgtctt tctttgtcat attcgtacct ttcccagttt 140
gcgatgccca agaagcaacc tttctgttgg aaacatctag tgctcaattc attatttcaa attgttgaaa 210
tcttcaagga gcagtttatt gtgcgccctt gttgattgct cttgtttatt tacaagtggg gatttaggcg 280
ctgggttcat atatgttctt tgtttctgcc aggtttcctt gttaggctct agttttgctc gttgtttact 350
gtaaattccc atgtatatta agtttgtgca gtgcgctgtc agcttggcaa atgggctttt cttttcctga 420
tcgaaaaaaa aacaaacctg tgattaacaa ggggaaatta ccttcactgt gcctattggc atcattccaa 490
gttattcatg ctttagttcc tagccattaa cttaagatag aaataagatc ctgccagttc actagttttc 560
ttgtcagttt gatatatact ttgatcttgt cttttctggt tgttttcctt aattttgtct gtgttgttct 630
agtgcttaat tagttgcctc tcagttcttc acattctatg tttttgagta aaactctgtc catatggccg 700
ttctgttatg tatctcagca atctgcttat attttttttt tgcatcccca cacaattcta tttgacgttg 770
taaaaaagaa gagagataac tagataagcg caactgatgc tctatactta gtttcacggt tcttggttta 840
atgcctaaaa attgtgcact atcagtactt caattatgtt atctagtagt gttttgtctc ctctgaataa 910
tgcctgccat tcttatgctg ctctactgct gagaaaacta cactatttga attactgatt gctaattatg 980
tttcgattaa cagcataaag gaacattttg ttgatgttct aatggagtca aaaggtggca aaaagaagtc 1050
tagcagtagt agttccatgt acgaagctcc cctaggctac aagattgagg acgttcgccc agccggagga 1120
atcaagaagt tccagtctgc tgcttactcc aacgtaagca ccaaaagatt taagcagcat actttaaata 1190
acaaaaatat ttacatttat gaactctggt ttctaatgct tatttggttt atcttttcct ttgcagtgcg 1260
ctcgcaagcc atcctgatat tccttcacat gcctttcccc gtagagtagg aatttcctgc aatttttcct 1330
ttgttttgtt gcaataaatc tcccttggct ggctgctttc tcaatctctt cccttgtctt ctccgatctc 1400
ttctttcctg tcttgtccac tgctccttgc aacacttgat caaatgtcga tgtccttggc tgattcttgg 1470
ggctctgccc ctgcctcgcc cattgggttt gaaggctatg agaagcgcct tgagataaca ttgtctgatg 1540
cgcccgtctt tgtggacccc tgtggtcgcg gccttcgtgc cctctcacgt gagcagatcg actcgttctt 1610
ggatcttgca aagtgtacca ttgtatccca tctttcgaac aagcacttcg actcatatgt gctttcagag 1680
tcgagccttt ttgtttatcc ccacaaggtt gttctgaaga cctgtggtac aacaaagctt ctgctctcca 1750
ttcctcgcat ccttgagctt gctgcagagt tgtcactgcc tgttctatca gtgaagtact ctcgtgggat 1820
gttcatcttc cctggagcgc agccatcacc gcatcgcagc ttcttggagg aggtttctgt gctcaacagc 1890
ttctttggtg gcctcaagtc aggtggcaat gcttatgtca ttggtgatgc attcaagccc aagaagaagt 1960
ggcatgtcta ctatgccaca gaagagcctg agcagcccat ggttacactc gagatgtgca tgactgggct 2030
ggatgctaag aaagctgagg tgttcttcaa ggattccact gatggctcct gctcatcagc taaggagatg 2100
actatgttct ctgggatttc tgaaatcatc cctgagatgg agatttgtga ctttgagttt gacccgtgtg 2170
ggtactcaat gaatggcatt tatggtcccg ccgtctccac gatccatgtc actcctgagg aaggtttcag 2240
ctatgcaagc tatgaagcaa tgaacttcaa tcctagctcc ttggtctacg atgatttgat caagaaggtc 2310
ctggcttgtt tctgcccttc agacttttcg gtcgctgtta ccatcttcgg tgggcatggt tttgccaaat 2380
catgggcaaa aggtgcagag gttgattcct acatgtgcga tgatcttgtt gagcaagagc ttcctggtgg 2450
cggtgtgctg atgtatcaga gttttactgc tgttactcct ggtgctgtgt caccgaggtc gaccttggat 2520
ggctggaaca gcgatggagc agagatggtt gcgaagagca aagagatgag tgtctgctgg gaaggagaga 2590
 
aggcggcgaa gaagaaagat gcagatgcct gagaaatcgg cagttcttca acctcaagtt gcaagtttga 2660
ttcatctgaa gtttctgaga cgccttattt ggttctgctc ttgcagtttc ggttaagcag ctggctgggc 2730
taccggcaaa gcatatgtta cttgttcatt gttcttttat atctgtctgc ccaaataagt gctctagatg 2800
gtttgctgcg tctgccatag tgagcaattc agattgtagt agaattctta tctgttgccg atgctcttta 2870
agggagaatg atatgtaact cgattatttt caataactat tgcaacttcc ataattttct gtccattggt 2940
tatgtttgtt tg 2952
<210> 18
   <211> 392
   <212> PRT
   <213> Oryza sativa
   <400> SEQ ID NO: 18 
Met Ser Met Ser Leu Ala Asp Ser Trp Gly Ser Ala Pro Ala Ser Pro Ile Gly
1               5                   10                  15

Phe Glu Gly Tyr Glu Lys Arg Leu Glu Ile Thr Leu Ser Asp Ala Pro Val Phe
    20                  25                  30                  35

Val Asp Pro Cys Gly Arg Gly Leu Arg Ala Leu Ser Arg Glu Gln Ile Asp Ser
            40                  45                  50

Phe Leu Asp Leu Ala Lys Cys Thr Ile Val Ser His Leu Ser Asn Lys His Phe
55                  60                  65                  70

Asp Ser Tyr Val Leu Ser Glu Ser Ser Leu Phe Val Tyr Pro His Lys Val Val
        75                  80                  85                      90

Leu Lys Thr Cys Gly Thr Thr Lys Leu Leu Leu Ser Ile Pro Arg Ile Leu Glu
                95                  100                 105

Leu Ala Ala Glu Leu Ser Leu Pro Val Leu Ser Val Lys Tyr Ser Arg Gly Met
    110                 115                 120                 125

Phe Ile Phe Pro Gly Ala Gln Pro Ser Pro His Arg Ser Phe Leu Glu Glu Val
            130                 135                 140

Ser Val Leu Asn Ser Phe Phe Gly Gly Leu Lys Ser Gly Gly Asn Ala Tyr Val
145                 150                 155                 160

Ile Gly Asp Ala Phe Lys Pro Lys Lys Lys Trp His Val Tyr Tyr Ala Thr Glu
        165                 170                 175                     180

Glu Pro Glu Gln Pro Met Val Thr Leu Glu Met Cys Met Thr Gly Leu Asp Ala
                185                 190                 195

Lys Lys Ala Glu Val Phe Phe Lys Asp Ser Thr Asp Gly Ser Cys Ser Ser Ala
    200                 205                 210                 215

Lys Glu Met Thr Met Phe Ser Gly Ile Ser Glu Ile Ile Pro Glu Met Glu Ile
            220                 225                 230

Cys Asp Phe Glu Phe Asp Pro Cys Gly Tyr Ser Met Asn Gly Ile Tyr Gly Pro
235                 240                 245                 250

Ala Val Ser Thr Ile His Val Thr Pro Glu Glu Gly Phe Ser Tyr Ala Ser Tyr
        255                 260                 265                     270

Glu Ala Met Asn Phe Asn Pro Ser Ser Leu Val Tyr Asp Asp Leu Ile Lys Lys
                275                 280                 285

Val Leu Ala Cys Phe Cys Pro Ser Asp Phe Ser Val Ala Val Thr Ile Phe Gly
    290                 295                 300                 305

Gly His Gly Phe Ala Lys Ser Trp Ala Lys Gly Ala Glu Val Asp Ser Tyr Met
            310                 315                 320

Cys Asp Asp Leu Val Glu Gln Glu Leu Pro Gly Gly Gly Val Leu Met Tyr Gln
 
325                 330                 335                 340

Ser Phe Thr Ala Val Thr Pro Gly Ala Val Ser Pro Arg Ser Thr Leu Asp Gly
        345                 350                 355                 360

Trp Asn Ser Asp Gly Ala Glu Met Val Ala Lys Ser Lys Glu Met Ser Val Cys
                365                 370                 375

Trp Glu Gly Glu Lys Ala Ala Lys Lys Lys Asp Ala Asp Ala
    380                 385                 390
<210> 19
   <211> 2523
   <212> DNA
   <213> Populus deltoides
   <220>
   <400> SEQ ID NO: 19 
ttaaccctat atttctctca gaactcacat aaaaaatatt gcgagtttag ggtcataaaa atctctatca 70
tcacaaacaa atatagggtt tgttgatttg gtggctgttt tcgccgtctt tgctttcaaa attcaatctt 140
gacctgtttc ttttatgctt tttgaatagg taatatatac attgtttgtt ttgcttgctt tggttttgta 210
atctggtgat cttgtttaat tctttttggc tttggtttgt aggttttttt ttttgaacyt gagaaagagg 280
ggggttttca arggagacta gcttttcaag awttgatata ttttattggt aagtttttnt tttccgtttt 350
ttggtaatag ttgcatggat tcttggtgta gatgttgatt tggacttgtt tttgtattgt tgtcctttat 420
gttttatgtg tatgttaatt gttctgatcc ttgatataat gtaaatctga ggcttttatt tttatggatt 490
cgttaaggtg tatggacttg gtttatggtt ttggtcttga aaaaaaaatt aggcttagca agttttcttc 560
tgagaataac ttattttatt ttattattta aaattaaatg gtgtttggga atttgaattc tttctctttg 630
gtatttttttc tatccttgtg aatctgttta gtcttttaaa ttcaatattt tattttattt tacttatgat 700
ttggtgattg attagttatt tttgtagctt gtattgtctg ttgattttta atctccaata tgtcttactt 770
ctcagttttt ttcaatttgc tattggaatt ttgctattat ttttaagatt gtagactagt ttgctttgag 840
ttttgtgcta attgcaatcc ttcttgtgtg attcttacag gactgaatga tctaatggag tctaaaggtg 910
gcaagaagaa gtctagtagt agtagtagta gtaaatcctt atgttaagaa gctcccctcg gttacagcat 980
tgaagacctc agacctgctg gtggaatcaa gaagttcaga tctgctgcat actccaacgt gagtttgaag 1050
ttaatcgatc tgtttttcct ttggcatttc attgtgcgac tggtttccta atcttgtgtc atgtttattt 1120
gtgcagtgcg ttcgaaaacc atcctgagat tttccaagtg ttgacataac cccattttag ctatttcgca 1190
cgctcagttg tctttaktct gtttttctgt tctgctttcc tcgttctctt gtactctttg ctgcactttt 1260
catttgttga ctgtgaggtc agatggcgct gccagtctct gcaatcggat ttgaaggtta cgaaaaaagg 1330
cttgaaatat ctttcctaga gcctggcttc ttttctgacc ctgaagggaa gggcctgagg tctttgtcca 1400
aggctcaatt ggacgagatt ctcagaccag ctgaatgtac tattgttgat tcgctatcaa atgaccaggt 1470
tgattcttat gtcctgtcgg aatccagtct ctttgtgtac ccttacaaag ttattatcaa aacatgtggg 1540
actaccaaac tgcttctctc gataccggtg attcttaagc tcgctgatgc cctttcactc actgtatgtt 1610
ctgtgaggta tactcgtggg agctttctat gccctggggc tcagccattt ccacaccgca acttctgtga 1680
ggaggtagct gtccttgacg atcacttcag taaacttggt ttaaacagtg tggcatatgt gatgggtggt 1750
cttgacaaaa ctcagaaatg gcatgtttac tctgcctctg ccgatataga gagccattct ggccctgttt 1820
acactctgga aatgtgcatg actggtttgg gcaggaaaca agcatctgtt ttctacaaaa cacattccag 1890
ttcagctgct gcgatgactg aggattccgg tataaggaaa atccttccac agtctgagat ctgtgatttt 1960
 
gattttgacc catgtggtta ctctatgaat gccattgaag ggagtgcaat ttccacaatc cacgtcactc 2030

cagaagatgg tttcagctat gcaagttttg aggctgtggg ctatgatctt caagatttga atttgagtcg 2100

gctgcttgaa agggtcttgg cttgctttga accgaccatg ttctccgttg ccttgcattc taatatcaag 2170

ggtgccgaac ttagagcaaa gtttcccctg gacgtggaag gttactctgg cggaggaggg aactatgaaa 2240

tgcttgggaa aggtggatcg atcatctacc acagctttgc aaggactgga ggcagtgcat ctcccaggtc 2310

tatcctgaaa tgttgttgga gtgaggatga gaaggacgag gaagctgaag agaagtagtt cttttcagct 2380

atttgttttt tccttttatt ttttccttga ataaatgtca tggggttatg attctgagtt cttgaggcat 2450

ttgtccatgc ctcgtgtctt tcattatcga tttagttttg acttcggatt aataaagggg tttgtgttat 2520

cag 2523
<210> 20
   <211> 24
   <212> DNA
   <213> Artificial sequence
   <220>
   <400> SEQ ID NO: 20
   ATCCCATGGA TTCGGCCTTG CCTG 24
<210> 21
   <21i> 34
   <212> DNA
   <213> Artificial sequence
   <220>
   <400> SEQ ID NO: 21
   GTCTAGACTA CTCCTTCTCT TCTTTCTCTT CATC 34
<210> 22
   <211> 25
   <212> DNA
   <213> Artificial sequence
   <220>
   <400> SEQ ID NO: 22
   TATCGTTTTA CTTCACTGGT CGGTG 25
<210> 23
   <211> 20
   <212> DNA
   <213> Artificial sequence
   <220>
   <400> SEQ ID NO: 23
   AATCTCACCA ACCCAACTCC 20

## Claims

1. A transgenic plant wich contains an exogenous S-adenosylmethionine decarboxylase enzyme encoding nucleic acid molecule, wherein said plant Is a gymnosperm having reduced lignin content and/or reduced gualacyl lignin content as compared to a non-transgenic plant lacking said exogenous S-adenosylmethionine decarboxylase enzyme encoding nucleic acid molecule.

2. The transgenic plant of claim 1, obtained by transforming a plant with an expression vector containing said S-adenosylmethionine decarboxylase enzyme encoding nucleic add molecule, under the control of a promoter or promoters capable of functioning in plants.

3. The transgenic plant of claim 1, where said promoter is a cambium/xylem preferred promoter.

4. The transgenic plant of claim 1, wherein said nucleic acid molecule has:
(a) a nucleotide sequence comprising SEQ ID Nos.: 1, 3, 5, 7, 9, 11, 13, 15, 17, or 19;
(b) a nucleotide sequence coding for a protein with S-adenosylmethionine decarboxylase activity which hybridizes under stringent conditions with the base sequence of (a); or
(c) a nucleotide sequence of (a) or (b) with one or more bases deleted, substituted, inserted, or added.

5. The transgenic plant of claim 1, wherein nucleic acid molecule encodes the S-adenosylmethionine decarboxylase enzyme set forth by SEQ ID No: 2.

6. The transgenic plant of claim 1, with reduced lignin content.

7. The transgenic plant of claim 1, with an increased syringyl lignin/guaiacyl lignin ratio.

8. The transgenic plant of claim 1, with reduced guaiacyl lignin content.

9. The transgenic plant of claim 1, wherein said plant is a woody tree.

10. The transgenic plant of claim 9, wherein said woody tree is a *Pinus* plant.

11. A part of the transgenic plant of claim 1, selected from the group consisting of a leaf, a stem, a flower, an ovary, a fruit, a seed and a callus wherein said part contains an exogenous S-adenosylmethionine decarboxylase enzyme encoding nucleic acid molecule and has a reduced lignin content and/or reduced guaiacyl lignin content as compared to a non-transgenic plant part lacking said exogenous S-adenosylmethionine decarboxylase enzyme encoding nucleic acid molecule.

12. A method for producing a transgenic plant according to any of the preceding claims, comprising transforming a cell of a plant with a nucleic add molecule comprising an exogenous S-adenosylmethionine decarboxylase enzyme encoding sequence under the control of a promoter capable of functioning in plants, and culturing said cell to produce a transgenic plant.

13. The method of claim 12, wherein said S-adenosylmethionine decarboxylase enzyme encoding sequence comprises a base sequence of (a), (b), or (c) below:
(a) a nucleotide sequence comprising SEQ ID Nos.: 1, 3, 5, 7, 9, 11, 13, 15, 17, or 19;
(b) a nucleotide sequence coding for a protein with S-adenosylmethionine decarboxylase activity which hybridizes under stringent conditions with the base sequence of (a) above; or
(c) a nucleotide sequence of (a) or (b) with one or more bases deleted, substituted, inserted, or added.

14. The method according to claim 12, wherein said S-adenosylmethionine decarboxylase enzyme encoding sequence encodes the S-adenosylmethionine decarboxylase enzyme set forth by SEQ ID No: 2.

## Patentansprüche

1. Transgene Pflanze, die ein für ein exogenes S-Adenosylmethionindecarboxylaseenzym codierendes Nucleinsäuremolekül enthält, wobei die Pflanze ein Gymnosperm ist und verglichen mit einer nicht transgenen Pflanze ohne das für das exogene S-Adenosylmethionindecarboxylaseenzym codierende Nucleinsäuremolekül einen herabgesetzten Ligningehalt und/oder herabgesetzten Guaiacylligningehalt aufweist.

2. Transgene Pflanze nach Anspruch 1, erhalten durch Transformation einer Pflanze mit einem Expressionsvektor, der das für das S-Adenosylmethionindecarboxylaseenzym codierende Nucleinsäuremolekül enthält, unter der Steuerung eines oder mehrerer in Pflanzen funktionsfähigen Promotoren.

3. Transgene Pflanzen nach Anspruch 1, wobei der Promotor ein cambium-/xylembevorzugter Promotor ist.

4. Transgene Pflanze nach Anspruch 1, wobei das Nucleinsäuremolekül aufweist:
(a) eine Nucleotidsequenz umfassend SEQ ID Nr.: 1, 3, 5, 7, 9, 11, 13, 15, 17 oder 19;
(b) eine Nucleotidsequenz, die für ein Protein mit S-Adenosylmethionindecarboxylase-Aktivität codiert und die unter stringenten Bedingungen mit der Basensequenz unter (a) hybridisiert; oder
(c) eine Nucleotidsequenz unter (a) oder (b), bei der eine oder mehrere Basen deletiert, substituiert, inseriert oder addiert sind.

5. Transgene Pflanze nach Anspruch 1, wobei das Nucleinsäuremolekül für das S-Adenosylmethionindecarboxylaseenzym gemäß SEQ ID Nr. 2 codiert.

6. Transgene Pflanzen nach Anspruch 1 mit herabgesetztem Ligningehalt.

7. Transgene Pflanze nach Anspruch 1 mit erhöhtem Syringyllignin-/Guaiacyllignin-Verhältnis.

8. Transgene Pflanzen nach Anspruch 1 mit herabgesetztem Guaiacylligningehalt.

9. Transgene Pflanze nach Anspruch 1, wobei die Pflanze ein Holzgewächs ist.

10. Transgene Pflanze nach Anspruch 9, wobei das Holzgewächs eine *Pinus-*Pflanze ist.

11. Teil einer transgener Pflanze nach Anspruch 1, ausgewählt aus der Gruppe bestehend aus einem Blatt, einem Stamm, einer Blüte, einem Ovarium, einer Frucht, einem Samen und einem Kallus, wobei der Teil ein für ein exogenes S-Adenosylmethionindecarboxylaseenzym codierendes Nucleinsäuremolekül enthält und verglichen mit einer nicht transgenen Pflanze ohne das für das exogene S-Adenosylmethionindecarboxylaseenzym codierende Nucleinsäuremolekül einen herabgesetzten Ligningehalt und/oder herabgesetzten Guaiacylligningehalt aufweist.

12. Verfahren zur Herstellung einer transgenen Pflanze nach einem der vorhergehenden Ansprüche, umfassend das Transformieren einer Zelle einer Pflanze mit einem Nucleinsäuremolekül, umfassend ein exogenes S-Adenosylmethionindecarboxylaseenzym, das für eine Sequenz unter der Steuerung eines in Pflanzen funktionsfähigen Promotors codiert, und Kultivieren der Zelle zum Erzeugen einer transgenen Pflanze.

13. Verfahren nach Anspruch 12, wobei die für das S-Adenosylmethionindecarboxylaseenzym codierende Sequenz eine nachstehende Basensequenz unter (a), (b) oder (c) umfasst:
(a) eine Nucleotidsequenz umfassend SEQ ID Nr.: 1, 3, 5, 7, 9, 11, 13, 15, 17 oder 19;
(b) eine Nucleotidsequenz, die für ein Protein mit S-Adenosylmethionindecarboxylase-Aktivität codiert und die unter stringenten Bedingungen mit der Basensequenz unter (a) hybridisiert; oder
(c) eine Nucleotidsequenz unter (a) oder (b), bei der eine oder mehrere Basen deletiert, substituiert, inseriert oder addiert sind.

14. Verfahren nach Anspruch 12, wobei die für das S-Adenosylmethionindecarboxylaseenzym codierende Sequenz für das S-Adenosylmethionindecarboxylaseenzym in SEQ ID Nr. 2 codiert.

## Revendications

1. Plante transgénique contenant une molécule d'acide nucléique codant pour une enzyme S-adénosylméthionine décarboxylase exogène, dans laquelle ladite plante est une gymnosperme possédant une teneur en lignine réduite et/ou une teneur en guaiacyl-lignine réduite par comparaison à une plante non transgénique exempte de ladite molécule d'acide nucléique codant pour une enzyme S-adénosylméthionine décarboxylase exogène.

2. La plante transgénique de la revendication 1, obtenue par transformation d'une plante avec un vecteur d'expression contenant ladite molécule d'acide nucléique codant pour une enzyme S-adénosylméthionine décarboxylase, sous le contrôle d'un ou de plusieurs promoteurs capables de fonctionner dans des plantes.

3. La plante transgénique de la revendication 1, dans laquelle ledit promoteur est un promoteur préférentiel du cambium/xylème.

4. La plante transgénique de la revendication 1, dans laquelle ladite molécule d'acide nucléique possède :
(a) une séquence nucléotidique comprenant SEQ ID N° : 1, 3, 5, 7, 9, 11, 13, 15, 17 ou 19 ;
(b) une séquence nucléotidique codant pour une protéine à activité S-adénosylméthionine décarboxylase qui s'hybride dans des conditions rigoureuses avec la séquence de bases de (a) ; ou
(c) une séquence nucléotidique de (a) ou (b) dont une ou plusieurs bases ont fait l'objet d'une délétion, d'une substitution, d'une insertion ou d'une addition.

5. La plante transgénique de la revendication 1, dans laquelle la molécule d'acide nucléique code pour l'enzyme S-adénosylméthionine décarboxylase définie par SEQ ID N° : 2.

6. La plante transgénique de la revendication 1, possédant une teneur en lignine réd u ite.

7. La plante transgénique de la revendication 1, possédant un rapport syringyl-lignine/guaiacyl-lignine augmenté.

8. La plante transgénique de la revendication 1, possédant une teneur en guaiacyl-lignine réduite.

9. La plante transgénique de la revendication 1, dans laquelle ladite plante est un arbre ligneux.

10. La plante transgénique de la revendication 9, dans laquelle ledit arbre ligneux est une plante de type *Pinus.*

11. Partie de la plante transgénique de la revendication 1, choisie dans le groupe consistant en une feuille, une tige, une fleur, un ovaire, un fruit, une graine et un cal dans laquelle ladite partie contient une molécule d'acide nucléique codant pour une enzyme S-adénosylméthionine décarboxylase exogène et possède une teneur en lignine réduite et/ou une teneur en guaiacyl-lignine réduite par comparaison à une partie de plante non transgénique exempte de ladite molécule d'acide nucléique codant pour une enzyme S-adénosylméthionine décarboxylase exogène.

12. Méthode de production d'une plante transgénique selon l'une quelconque des revendications précédentes, comprenant la transformation d'une cellule d'une plante avec une molécule d'acide nucléique comprenant une séquence codant pour une enzyme S-adénosylméthionine décarboxylase exogène sous le contrôle d'un promoteur capable de fonctionner dans des plantes, et la mise en culture de ladite cellule pour produire une plante transgénique.

13. La méthode de la revendication 12, dans laquelle ladite séquence codant pour une enzyme S-adénosylméthionine décarboxylase comprend une séquence de bases de (a), (b) ou (c) ci-dessous :
(a) une séquence nucléotidique comprenant SEQ ID N° : 1, 3, 5, 7, 9, 11, 13, 15, 17 ou 19 ;
(b) une séquence nucléotidique codant pour une protéine à activité S-adénosylméthionine décarboxylase qui s'hybride dans des conditions rigoureuses avec la séquence de bases de (a) ci-dessus ; ou
(c) une séquence nucléotidique de (a) ou (b) dont une ou plusieurs bases ont fait l'objet d'une délétion, d'une substitution, d'une insertion ou d'une addition.

14. La méthode selon la revendication 12, dans laquelle ladite enzyme S-adénosylméthionine décarboxylase code pour l'enzyme S-adénosylméthionine décarboxylase définie par SEQ ID N° : 2.
